(19) Europäisches Patentamt
European Patent Office
Office européen des brevets

(11) EP 1 761 575 B1

(12) FASCICULE DE BREVET EUROPEEN

(45) Date de publication et mention de la délivrance du brevet:
**19.08.2009 Bulletin 2009/34**

(21) Numéro de dépôt: **05777194.1**

(22) Date de dépôt: **10.06.2005**

(51) Int Cl.:
***C08F 220/18*** *(2006.01)*

(86) Numéro de dépôt international:
**PCT/FR2005/001445**

(87) Numéro de publication internationale:
**WO 2006/003317 (12.01.2006 Gazette 2006/02)**

# (54) COPOLYMERE A GRADIENT, COMPOSITION LE COMPRENANT ET PROCEDE COSMETIQUE DE MAQUILLAGE OU DE SOIN

GRADIENTENCOPOLYMER, DIESES ENTHALTENDE ZUSAMMENZETZUNG UND EIN VERFAHREN ZUR KOSMETISCHE BEHANDLUNG ODER PFLEGE

GRADIENT COPOLYMER, COMPOSITION INCLUDING SAME AND COSMETIC MAKE-UP OR CARE METHOD

(84) Etats contractants désignés:
**AT BE BG CH CY CZ DE DK EE ES FI FR GB GR HU IE IS IT LI LT LU MC NL PL PT RO SE SI SK TR**

(30) Priorité: **11.06.2004 FR 0406369**
**09.07.2004 US 586313 P**

(43) Date de publication de la demande:
**14.03.2007 Bulletin 2007/11**

(73) Titulaire: **L'ORÉAL**
**75008 Paris (FR)**

(72) Inventeur: **FARCET, Céline**
**F-75012 Paris (FR)**

(74) Mandataire: **Dodin, Catherine**
**L'Oréal**
**River Plaza - D.I.P.I.**
**25-29 Quai Aulagnier**
**92665 Asnières-sur-Seine Cedex (FR)**

(56) Documents cités:
**EP-A- 1 428 493**
**EP-A- 1 428 495**
**EP-A- 1 428 496**
**US-A1- 2003 096 929**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 761 575 B1

## Description

La présente invention a trait à de nouvelles compositions cosmétiques ou dermatologiques topiques, comprenant des copolymères à gradient particuliers, de préférence solubles dans le milieu solvant de la composition qui peut comprendre des huiles et/ou des solvants cosmétiques; l'invention concerne également les copolymères particuliers.

Dans le domaine de la cosmétique, on cherche souvent à disposer de compositions permettant d'obtenir un dépôt notamment adhésif ou filmogène, sur les matières kératiniques considérées, telles que les cheveux, la peau, les cils ou les ongles.

En particulier, ces compositions peuvent apporter de la couleur (compositions de maquillage ou de coloration capillaire), de la brillance ou de la matité (compositions de soin ou de maquillage de la peau), des propriétés physiques telles que de la mise en forme (compositions capillaires notamment de coiffage), des propriétés de soin ou de protection (compositions de soin, par exemple d'hydratation ou de protection UV).

On recherche généralement une bonne rémanence et tenue dans le temps du dépôt cosmétique, ainsi qu'une bonne adhésion sur le support. En particulier, il est souhaitable que ce dépôt puisse résister aux agressions mécaniques telles que frottements, transferts par contact d'un autre objet; à l'eau, à la sueur, aux larmes, à la pluie, au sébum et aux huiles. Ceci est particulièrement vrai en maquillage, notamment dans le domaine des rouge à lèvres où l'on recherche la tenue prolongée de la couleur et de la brillance et le non transfert de la couleur; dans le domaine des fonds de teint, fards à paupières et poudres où l'on recherche la tenue de la couleur apportée, en maintenant le plus longtemps possible la matité du maquillage initial malgré la sécrétion de sébum et de sueur, ainsi que le non transfert. En outre, les compositions de maquillage doivent être confortables à porter et ne pas présenter une texture trop collante.

Pour concilier l'ensemble de ces propriétés, souvent antinomiques, au sein d'une même composition, il est possible d'employer un mélange de plusieurs polymères, de nature chimiques très différentes, chaque polymère apportant une des caractéristiques souhaitées. Toutefois, l'emploi d'un mélange de polymères ayant des natures chimiques différentes, pas forcément compatibles, peut générer des problèmes de démixtion au sein de la composition.

L'utilisation de polymères statistiques, par exemple de polymères acryliques conventionnels obtenus par polymérisation radicalaire classique par mélange statistique de monomères, ne permet pas de résoudre ces problèmes de manière satisfaisante. En effet, les polymères statistiques connus dans l'art antérieur présentent une dispersité en composition des chaînes polymériques, ce qui conduit également à une démixtion des polymères au sein de la formule.

La présente invention a pour but de pallier les inconvénients de l'art antérieur en proposant une composition cosmétique ou dermatologique comprenant des polymères particuliers, qui évitent les problèmes de demixtion au sein de la formule tout en permettant d'apporter les propriétés cosmétiques recherchées.

Un objet de la présente invention est donc un copolymère à gradient comprenant au moins deux monomères différents choisis tous les deux parmi l'acrylate d'isobornyle, le méthacrylate d'isobornyle, l'acrylate d'isobutyle, le méthacrylate d'isobutyle et l'acrylate d'éthyle-2-hexyle.

Un autre objet de l'invention est une composition cosmétique ou dermatologique comprenant ledit copolymère.

De manière avantageuse, les copolymères selon l'invention sont parfaitement solubles dans le milieu solvant de la composition qui peut comprendre des huiles et/ou des solvants cosmétiques.

Comme les copolymères à gradient selon l'invention présentent une faible dispersité en composition, toutes les chaînes présentant quasiment les mêmes structures, elles sont donc compatibles entre elles; il en résulte que les compositions cosmétiques comprenant ces copolymères ne présentent pas les inconvénients et limitations des compositions de l'art antérieur.

Notamment les copolymères selon l'invention présentent l'avantage de pouvoir facilement être mis en oeuvre dans les milieux cosmétiques organiques, notamment de type huileux ou solvant lipophile, tout en conservant des propriétés rhéologiques intéressantes.

Les copolymères selon l'invention sont des copolymères à gradient, qui comprennent au moins deux monomères différents choisis dans une liste donnée, et qui présentent de préférence une polydispersité en masse faible et de préférence une polydispersité en composition faible.

La polydispersité en masse peut être illustrée à l'aide de l'indice de polydispersité en masse (Ip) du copolymère, qui est égal au rapport de la masse moléculaire moyenne en poids (Mw) sur la masse moléculaire moyenne en nombre (Mn). Une faible dispersité en masse traduit des longueurs de chaînes approximativement identiques, ce qui est le cas pour les copolymères selon la présente invention.

De préférence, le copolymère à gradient selon l'invention possède un indice de polydispersité en masse inférieur ou égal à 3, de préférence compris entre 1,1 et 2,5, notamment entre 1,15 et 2,3, voire entre 1,2 et 2,0, ou 1,9 ou encore 1,8.

Par ailleurs, la masse moléculaire moyenne en poids du copolymère à gradient est de préférence comprise entre 2000 g/mol et 1 000 000 g/mol, notamment entre 3000 g/mol et 800 000 g/mol, et encore mieux entre 5000 g/mol et 500 000 g/mol.

De préférence, la masse moléculaire moyenne en nombre du copolymère à gradient est comprise entre 2000 g/mol et 1 000 000 g/mol, notamment entre 3000 g/mol et 800 000 g/mol, et encore mieux entre 5000 g/mol et 500 000 g/mol.

On détermine les masses moléculaires moyennes en poids (Mw) et en nombre (Mn) par chromatographie liquide par perméation de gel (GPC), éluant THF, courbe d'étalonnage établie avec des étalons de polystyrène linéaire, détecteur réfractométrique.

Le copolymère à gradient selon l'invention présente également préférentiellement une faible dispersité en composition. Ceci signifie que toutes les chaînes de copolymères ont une composition (c'est-à-dire un enchaînement de monomères) approximativement analogue et sont donc homogènes en composition. Afin de montrer que toutes les chaînes de copolymères ont une composition analogue, on utilisera avantageusement la chromatographie d'adsorption liquide (ou LAC) qui permet de séparer les chaînes de copolymères, non pas selon leur poids moléculaire mais selon leur polarité. Cette dernière reflète la composition chimique des polymères constituant le matériau, les monomères étant connus. On pourra se reporter à la publication Macromolecules (2001), 34, 2667 qui décrit la technique LAC.

On peut notamment définir la polydispersité en composition à partir de la courbe de chromatographie d'adsorption (LAC) (courbe représentant la proportion de polymères en fonction du volume d'élution): si l'on nomme "$V^{1/2}$ min" la valeur minimum du volume d'élution à mi-hauteur de la courbe, et "$V^{1/2}$ max" la valeur maximum du volume d'élution à mi-hauteur de la courbe, la polydispersité en composition est généralement considérée comme faible si la différence ($V^{1/2}$ max - $V^{1/2}$ min) est inférieure ou égale à 3,5, notamment comprise entre 1 et 2,8 et mieux entre 1,2 et 2,5.

Par ailleurs, la courbe LAC peut être définie par une courbe de Gauss de formule :

$$y = \frac{A}{w\sqrt{\frac{\pi}{2}}} \times e^{-2\frac{(x-x_0)^2}{w^2}} + y_o$$

dans laquelle :

- $X_0$ est la valeur de x (volume d'élution) au centre du pic
- w est égal à 2 fois la déviation standard de la distribution gaussienne (soit 2σ) ou encore correspond à approximativement à 0,849 fois la largeur du pic à mi-hauteur
- A représente la surface sous le pic
- $y_o$ est la valeur de y correspondant à $x_0$.

La dispersité en composition du copolymère selon l'invention peut également être définie par la valeur de w telle que définie ci dessus. De préférence, ladite valeur w est comprise entre 1 et 3, notamment entre 1,1 et 2,3 et encore mieux entre 1,1 et 2,0.

Les copolymères à gradient selon l'invention peuvent être obtenus par polymérisation vivante ou pseudo-vivante.

Pour mémoire, nous rappelons que la polymérisation vivante est une polymérisation pour laquelle la croissance des chaînes polymériques ne cesse qu'avec la disparition du monomère. La masse moyenne en nombre (Mn) croît avec la conversion. La polymérisation anionique est un exemple typique de polymérisation vivante. De telles polymérisations conduisent à des copolymères dont la dispersité en masse est faible c'est-à-dire à des polymères d'indice de polydispersité en masse (Ip) généralement inférieur à 2.

La polymérisation pseudo-vivante est, quant à elle, associée à la polymérisation radicalaire contrôlée. Parmi les principaux types de polymérisation radicalaire contrôlée, on peut citer :

- la polymérisation radicalaire contrôlée par des nitroxydes. On peut notamment se référer aux demandes de brevet WO96/24620 et WO00/71501 qui décrivent les outils de cette polymérisation et leur mise en oeuvre, ainsi qu'aux articles publiés par Fischer (Chemical Reviews, 2001, 101, 3581), par Tordo et Gnanou (J. Am. Chem. Soc. 2000, 122, 5929) et Hawker (J. Am. Chem. Soc. 1999, 121, 3904);
- la polymérisation par transfert d'atome radicalaire, notamment décrite dans la demande WO96/30421 et qui procède par l'insertion réversible sur un complexe organométallique dans une liaison de type carbone-halogène;
- la polymérisation radicalaire contrôlée par des dérivés soufrés de type xanthate, dithioesters, trithiocarbonates ou carbamates, telle que décrite dans les demandes FR2821620, WO98/01478, WO99/35177, WO98/58974, WO99/31144, WO97/01478 et dans la publication de Rizzardo & al. (Macromolecules, 1998, 31, 5559).

La polymérisation radicalaire contrôlée désigne des polymérisations pour lesquelles les réactions secondaires qui conduisent habituellement à la disparition des espèces propageantes (réaction de terminaison ou transfert) sont rendues très peu probables par rapport à la réaction de propagation grâce à un agent de contrôle des radicaux libres. L'imperfection de ce mode de polymérisation réside dans le fait que lorsque les concentrations en radicaux libres deviennent importantes

par rapport à la concentration en monomère, les réactions secondaires redeviennent déterminantes et tendent à élargir la distribution des masses.

Grâce à ces modes de polymérisation, les chaînes polymériques des copolymères à gradient selon l'invention croissent simultanément et donc incorporent à chaque instant les mêmes ratio de co-monomères. Toutes les chaînes possèdent donc les mêmes structures ou des structures proches, d'où une faible dispersité en composition. Ces chaînes possèdent également un indice de polydispersité en masse faible.

Les copolymères à gradient sont des copolymères présentant une évolution du ratio des différents monomères tout au long de la chaîne. La distribution dans les chaînes polymériques des co-monomères dépend de l'évolution pendant la synthèse des concentrations relatives des co-monomères. Les copolymères selon l'invention comprennent au moins deux monomères différents dont la concentration le long de la chaîne polymérique change graduellement et de façon systématique et prédictible.

Ceci signifie que toutes les chaînes polymériques ont au moins un monomère Mi pour lequel, quelque soit la position normalisée x sur la chaîne polymérique, il y a une probabilité non nulle de retrouver ce monomère Mi le long de la chaîne. Une des caractéristiques permettant de définir les copolymères à gradient est le fait qu'à tout instant de la polymérisation, toutes les chaînes sont soumises à la présence de l'ensemble de tous les monomères. Ainsi, dans le milieu réactionnel, la concentration de chaque monomère est toujours non nulle, à tout instant de la polymérisation.

Ceci permet de différencier les copolymères selon l'invention des polymères-blocs usuels dans lesquels l'évolution des monomères le long de la chaîne polymérique n'est pas systématique par exemple pour un dibloc AB, au sein de la séquence A, la concentration en l'autre monomère B est toujours nulle.

Dans le cas des polymères statistiques, l'évolution des monomères le long de la chaîne polymérique ne sera pas non plus graduelle, systématique et prédictible. Un polymère statistique obtenu par polymérisation radicalaire classique de deux monomères se distingue d'un copolymère à gradient, par la répartition des monomères qui n'est pas identique sur toutes les chaînes, et par la longueur desdites chaînes qui n'est pas identique pour toutes les chaînes.

Pour une description théorique des copolymères à gradient, il est possible de se reporter aux publications suivantes :

T. Pakula et al., Macromol. Theory Simul. 5, 987-1006 (1996) ;
A. Aksimetiev et al. J. of Chem. Physics 111, n°5 ;
M. Janco, J. Polym. Sci., Part A: Polym. Chem. (2000), 38(15), 2767-2778 ;
M. Zaremski et al., Macromolecules (2000), 33(12), 4365-4372 ;
K. Matyjaszewski & al., J. Phys. Org. Chem. (2000), 13(12), 775-786 ;
Gray, Polym. Prepr. (Am. Chem. Soc., Div. Polym. Chem.) (2001), 42(2), 337-338 ;
K. Matyjaszewski, Chem. Rev. (Washington, D. C.) (2001), 101(9), 2921-2990.

Parmi les copolymères à gradient, on peut distinguer les copolymères à gradient naturel, et les copolymères à gradient artificiel.

Un copolymère à gradient naturel est un copolymère à gradient synthétisé en batch à partir d'un mélange initial des co-monomères. La distribution dans la chaîne des divers monomères suit une loi déduite de la réactivité relative et des concentrations initiales de monomères. Ces copolymères constituent la classe la plus simple de copolymères à gradient car c'est le mélange initial qui définit la propriété finale de produit.

Un copolymère à gradient artificiel est un copolymère dont on fait varier la concentration en monomères durant la synthèse par un artifice de procédé. Dans ce cas, on passe d'un mélange de monomères à un autre dans la chaîne du fait d'un changement subit et brusque des monomères dans le milieu réactionnel (stripping du premier mélange ou addition d'au moins un nouveau monomère). Il peut même y avoir une disparition nette d'un ou plusieurs des monomères au profit d'un ou plusieurs autres.

La caractérisation expérimentale du gradient peut être apportée par la mesure en cours de polymérisation de la composition chimique du polymère. Cette mesure est faite de façon indirecte en déterminant l'évolution de la teneur des différents monomères à tout instant. Elle peut être faite par RMN et UV, par exemple. En effet, pour les polymères préparés par polymérisation vivante ou pseudo-vivante, la longueur des chaînes est linéairement liée à la conversion. En prélevant un échantillon de la solution de polymérisation, à différents instants de la polymérisation et en mesurant la différence de teneur en chaque monomère, on accède ainsi à la composition du gradient.

Dans le polymère à gradient, la distribution des compositions des chaînes est de préférence étroite. En particulier il n'existe pas de recouvrement entre le pic du copolymère à gradient et ceux des homopolymères respectifs. Ceci signifie que le matériau obtenu en gradient est constitué de chaînes polymères de même composition alors qu'en polymérisation statistique classique, différentes sortes de chaîne coexistent, y compris celles des homopolymères respectifs.

Il est possible de caractériser les copolymères à gradient par un vecteur caractéristique de chaque copolymère. En effet, sachant qu'il existe une infinité de polymères caractérisés par une composition chimique donnée, pour préciser

un polymère il est possible de décrire la répartition des monomères le long de la chaîne. Ce qui implique une description à plusieurs variables. Ce vecteur est un point de l'espace des compositions chimiques. Le terme exact est que G est un vecteur dont les coordonnées sont les concentrations des monomères le long de la chaîne polymérique. Ces concentrations sont définies par les règles des coefficients de réactivité de chacun des monomères, et donc sont liées à la concentration des monomères libres pendant la synthèse : du moment que le monomère n'est pas en concentration nulle dans le mélange réactionnel, il n'est pas en concentration nulle dans le polymère.
Il est donc possible de caractériser les copolymères à gradient par la fonction G(x) qui définit le gradient de composition :

$$\vec{G}(x) = \sum \overrightarrow{[Mi](x)}$$

dans laquelle :

- x désigne une position normalisée sur la chaîne polymère et
- [Mi](x) est la concentration relative, en cette position x, du monomère Mi exprimée en % en moles.

La fonction G(x) décrit donc localement la composition du copolymère à gradient.
Deux copolymères peuvent avoir une composition globale équivalente mais des répartitions locales des monomères très différentes, donc des gradients différents.
Par exemple, dans le cas d'un dibloc AB (50/50), la fonction [A] vaut 1 jusqu'à x=1/2 puis 0 ensuite.
Les facteurs déterminant le gradient sont d'une part les coefficients de réactivité relatifs de chaque monomère (appelé $r_i$ pour le monomère Mi) qui dépendent principalement du type de procédé de synthèse employé (homogène, dispersé) et des solvants, et d'autre part les concentrations initiales de chacun des monomères, ainsi que les ajouts éventuels de monomères au cours de la polymérisation.
Le copolymère à gradient selon l'invention comprend au moins deux monomères différents, qui sont tous les deux choisis parmi l'acrylate d'isobornyle, le méthacrylate d'isobornyle, l'acrylate d'isobutyle, le méthacrylate d'isobutyle et l'acrylate d'éthyle-2-hexyle. Dans la suite de la présente description, ces cinq monomères seront appelés "monomères sélectionnés".
Le copolymère selon l'invention peut donc comprendre de 1 à 99% en poids par rapport au poids du copolymère final, notamment de 2-98% en poids, de préférence de 5-95% en poids, voire de 30 à 70% en poids, d'un premier monomère choisi parmi les "monomères sélectionnés" et 1 à 99% en poids par rapport au poids du copolymère final, notamment de 2-98% en poids, de préférence de 5-95% en poids, voire de 30 à 70% en poids, d'un second monomère, différent dudit premier monomère, et choisi parmi les "monomères sélectionnés".
Le copolymère selon l'invention peut également comprendre, de manière optionnelle, de 1 à 50% en poids, notamment de 5 à 40% en poids, voire de 10 à 35% en poids, par rapport au poids du copolymère final, d'un troisième monomère, différent des premier et second monomères, et choisi parmi les "monomères sélectionnés".
La quantité totale de monomères choisis parmi les monomères sélectionnés, dans le copolymère final, est de préférence comprise entre 50 et 100% en poids inclus, notamment de 60 à 98% en poids, voire de 70 à 97% en poids, encore mieux de 80 à 96% en poids, préférentiellement de 90 à 95% en poids par rapport au poids du copolymère final.
On préfère tout particulièrement les copolymères comprenant :

- de l'acrylate d'isobornyle et du méthacrylate d'isobornyle;
- de l'acrylate d'isobornyle et de acrylate d'isobutyle;
- de l'acrylate d'isobornyle et du méthacrylate d'isobutyle;
- de l'acrylate d'isobornyle et de l'acrylate d'éthyle-2-hexyle;
- du méthacrylate d'isobornyle et de l'acrylate d'isobutyle;
- du méthacrylate d'isobornyle et du méthacrylate d'isobutyle;
- du méthacrylate d'isobornyle et de l'acrylate d'éthyle-2-hexyle;
- de l'acrylate d'isobornyle, du méthacrylate d'isobornyle et de l'acrylate d'éthyle-2-hexyle;
- de l'acrylate d'isobornyle, du méthacrylate d'isobornyle et de l'acrylate d'isobutyle;
- de acrylate d'isobornyle, du méthacrylate d'isobutyle et de l'acrylate d'éthyle-2-hexyle;
- de l'acrylate d'isobornyle, du méthacrylate d'isobutyle et de l'acrylate d'isobutyle; ou
- du méthacrylate d'isobornyle, du méthacrylate d'isobutyle et de l'acrylate d'isobutyle.

Le copolymère selon l'invention peut en outre comprendre au moins un monomère additionnel autre que ceux choisis parmi les monomères sélectionnés.
Ce monomère additionnel, ou mélange de monomères additionnels, peut être présent en une quantité de 0 à

50% en poids, notamment de 2 à 40% en poids, voire de 3 à 30% en poids, encore mieux de 4 à 20% en poids, préférentiellement de 5 à 10% en poids, par rapport au poids du copolymère final.

Ce ou ces monomères additionnels peuvent être choisis parmi, seul ou en mélange, les monomères suivants, ainsi que leurs sels, à l'exclusion bien sûr des monomères sélectionnés ci-dessus mentionnés :

- (i) les (méth)acrylates de formule $CH_2$=CHCOOR ou $CH_2$=C($CH_3$)COOR dans laquelle R représente :

  - un groupe alkyle linéaire ou ramifié, comprenant 1 à 30 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I est F), les groupes -NR4R5, où R4 et R5 identiques ou différents représentent l'hydrogène ou un groupe alkyle linéaire ou ramifié, en C1 à C6 ou un groupe phényle; et/ou les groupes polyoxyalkylènes, notamment polyoxyéthylène et/ou polyoxypropylène, ledit groupe polyoxyalkylène étant constitué par la répétition de 5 à 30 motifs oxyalkylène;
  - un groupe cycloalkyle en C3 à C12, ledit groupe cycloalkyle pouvant comporter dans sa chaîne un ou plusieurs hétéroatomes choisis parmi O, N, S et/ou P, et/ou pouvant être éventuellement substitué par un ou plusieurs substituants choisis parmi -OH et les atomes d'halogène (Cl, Br, I et F);
  - un groupe aryle en C4 à C20 ou aralkyle en C5 à C30 (groupe alkyle en C1 à C8);

  notamment R peut être un groupe méthyle, éthyle, propyle, isopropyle, n-butyle, isobutyle, tertiobutyle, hexyle, éthylhexyle, octyle, lauryle, isooctyle, isodécyle, dodécyle, cyclohexyle, t-butylcyclohexyle, stéaryle, éthyl-2-perfluorohexyle, 2-hydroxyéthyle, 2-hydroxybutyle, 2-hydroxypropyle, méthoxyéthyle, éthoxyéthyle, méthoxypropyle, isobornyle, phényle, 2-phényl-éthyle, t-butylbenzyle, benzyle, furfurylméthyle ou tétrahydrofurfurylméthyle, méthoxy-polyoxyéthylène (ou POE-méthyle); POE-béhényle, trifluoroéthyle; diméthylaminoéthyle, diéthylaminoéthyle, diméthylaminopropyle.

- (ii) les (méth)acrylamides de formule $CH_2$=CHCON$R_4R_5$ ou $CH_2$=C($CH_3$)CON$R_4R_5$
  dans laquelle R4 et R5, identiques ou différents, représentent un atome d'hydrogène ou :

  a) un groupe alkyle, linéaire ou ramifié, comportant de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R4R5), où R4 et R5 identiques ou différents représentent un groupe alkyle en C1 à C6 ou un groupe phényle ; et notamment un groupe méthyle, éthyle, propyle, isopropyle, n-butyle, isobutyle, tertiobutyle, hexyle, isohexyle, cyclohexyle, éthylhexyle, octyle, isooctyle, décyle, isodécyle, cyclodécyle, dodécyle, cyclododécyle, isononyle, lauryle, t-butylcyclohexyle, stéaryle; éthyl-2-perfluorohexyle; ou un groupe hydroxyalkyle en C1-4 tel que 2-hydroxyéthyle, 2-hydroxybutyle et 2-hydroxypropyle; ou un groupe alcoxy(C1-4)-alkyle(C1-4) tel que méthoxyéthyle, éthoxyéthyle et méthoxy-propyle,
  b) un groupe cycloalkyle en C3 à C12, tel que le groupe isobomyle, ou un groupe hétérocycloalkyle (alkyle de 1 à 4 atomes de carbone), tel que furfurylméthyle ou tétrahydrofurfurylméthyle,
  c) un groupe aryle en C4 à C20 tel que le groupe phényle,
  d) un groupe aralkyle en C5 à C30 (groupe alkyle en C1 à C8) tel que 2-phényl éthyle, t-butylbenzyle ou benzyle,

- (iii) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylamidopropanesulfonique; et les sels de ceux ci;

- (iv) les éthers de vinyle de formule $R_6$O-CH=$CH_2$ ou les esters de vinyle de formule : $R_6$-COO-CH=$CH_2$
  dans laquelle $R_6$ représente un groupe alkyle linéaire ou ramifié, comprenant de 1 à 22 atomes, ou un groupe alkyle cyclique comportant de 3 à 6 atomes de carbone et/ou un groupe aromatique, par exemple de type benzénique, anthracénique, et naphtalénique ;

- (v) les composés vinyliques de formules: CH2=CH-R9, CH2=CH-CH2-R9 ou CH2=C(CH3)-CH2-R9
  dans lesquelles R9 est un groupe hydroxyle, halogène (Cl ou F), NH2, OR10 où R10 représente un groupe phényle ou un groupe alkyle en C1 à C12 (le monomère est un éther de vinyle ou d'allyle); acétamide (NHCOCH3); un groupe OCOR11 où R11 représente un groupe alkyle de 2 à 12 carbones, linéaire ou ramifié (le monomère est un ester de vinyle ou d'allyle); ou un groupe choisi parmi :

- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R4R5), où R4 et R5 identiques ou différents représentent un groupe alkyle en C1 à C6 ou un groupe phényle ;
- un groupe cycloalkyle en C3 à C12 tel que isobomyle, cyclohexane,
- un groupe aryle en C3 à C20 tel que phényle,
- un groupe aralkyle en C4 à C30 (groupe alkyle en C1 à C8) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,

- (vi) le styrène et ses dérivés, notamment tels que le méthylstyrène, le chlorostyrène ou le chlorométhylstyrène;

- (vii) les monomères à insaturation éthylénique comprenant un ou plusieurs atomes de silicium tels que le méthacryloxypropyl triméthoxy silane, le méthacryloxypropyl tris(triméthylsiloxy)silane;

- ainsi que leurs sels et leurs mélanges.

Parmi ces monomères additionnels, on peut tout particulièrement citer les (méth)acrylates de méthyle, de n-propyle, d'isopropyle, de n-butyle, de t-butyle, de cyclohexyle, de méthoxyéthyle, d'éthoxyéthyle, de trifluoroéthyle, de diméthylaminoéthyle, de diéthylaminoéthyle, de 2-hydroxypropyle, de 2-hydroxyéthyle; l'acide acrylique, l'acide méthacrylique, le (méth)acrylamide, le méthacryloxypropyl triméthoxy silane, le méthacryloxypropyl tris(triméthylsiloxy)silane; ainsi que leurs sels; et leurs mélanges.

Comme monomères additionnels, on peut également employer des macromonomères carbonés ou siliconés ayant au moins un groupe terminal polymérisable. Il s'agit de tout polymère, notamment oligomère, comportant sur une seule de ses extrémités un groupe terminal, notamment polymérisable, apte à réagir lors de la réaction de polymérisation avec les monomères considérés, pour former les chaînes latérales du polymère; ledit groupe terminal peut être avantageusement un groupe à insaturation éthylénique susceptible de se polymériser par voie radicalaire avec les monomères constituant le squelette. Ledit macromonomère permet de former les chaînes latérales du copolymère. Le groupe polymérisable du macromonomère peut être avantageusement un groupe à insaturation éthylénique susceptible de se polymériser par voie radicalaire. Ledit groupe terminal polymérisable peut être en particulier un groupe vinyle ou (méth) acrylate. Parmi les macromonomères additionnels susceptibles d'être employés, on peut notamment citer, seuls ou en mélange, ainsi que leurs sels, :

- (i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate; les macromonomères de poly(acrylate de stéaryle) ou de poly (méthacrylate de stéaryle) à extrémité mono(méth)acrylate. De tels macromonomères sont notamment décrits dans les brevets EP895467 et EP96459.
- (ii) les polyoléfines ayant un groupe terminal à insaturation éthylénique, en particulier ceux ayant un groupement terminal (méth)acrylate. Comme exemple de telles polyoléfines, on peut citer en particulier les macromonomères suivants, étant entendu qu'ils ont un groupe terminal (méth)acrylate: les macromonomères de polyéthylène, les macromonomères de polypropylène, les macromonomères de copolymère polyéthylène/polypropylène, les macromonomères de copolymère polyéthylène/polybutylène, les macromonomères de polyisobutylène; les macromonomères de polybutadiène; les macromonomères de polyisoprène; les macromonomères de polybutadiène; les macromonomères de poly(éthylène/butylène)-polyisoprène. De tels macromonomères sont en particulier décrits dans US 5,625,005 qui mentionne des macromonomères éthylène/butylène et éthylène/propylène à groupement terminal réactif (méth)acrylate. On peut en particulier citer le méthacrylate de poly(éthylène/butylène), tel que celui commercialisé sous la dénomination Kraton Liquid L-1253 par Kraton Polymers.
- (iii) les polydiméthylsiloxanes à groupement terminal mono(méth)acrylate, et notamment ceux de formule (IIa) suivante :

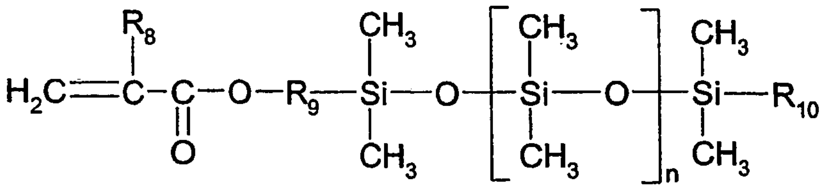

(IIa)

dans laquelle :

- $R_8$ désigne un atome d'hydrogène ou un groupement méthyle; de préférence méthyle;
- $R_9$ désigne un groupe hydrocarboné divalent, linéaire ou ramifié, de préférence linéaire, ayant de 1 à 10 atomes de carbone et contenant éventuellement une ou deux liaisons éther -O-; de préférence éthylène, propylène ou butylène;
- $R_{10}$ désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

Comme macromonomères siliconés, on peut en particulier citer les monométhacryloyloxypropyl polydiméthylsiloxanes tels que ceux commercialisés sous la dénomination PS560-K6 par UCT (United Chemical Technologies Inc.) ou sous la dénomination MCR-M17 par Gelest Inc.

Parmi les sels, on peut citer ceux obtenus par neutralisation des groupements acides à l'aide de base minérales telles que LiOH, NaOH, KOH, Ca(OH)2, NH4OH ou Zn(OH)2; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)-propylamine.

On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

De préférence, le copolymère à gradient selon l'invention comprend au moins un monomère ayant une Tg inférieure ou égale à 20°C, notamment comprise entre -150°C et 20°C, plus particulièrement comprise entre -130°C et 18°C, et encore mieux comprise entre -120°C et 15°C, ou un mélange de tels monomères.

Ces monomères peuvent être choisis parmi les "monomères sélectionnés" ci-dessus mentionnés et/ou parmi les monomères additionnels.

Ce ou ces monomères de Tg $\leq$ 20°C peuvent être présents à raison de 1 à 99% en poids, notamment 10-90% en poids, encore mieux 20-80% en poids, voire 25-75% en poids, par rapport au poids total du copolymère.

De préférence, le copolymère selon l'invention comprend donc également au moins un monomère ayant une Tg supérieure ou égale à 20°C, notamment comprise entre 25 et 150°C, plus particulièrement comprise entre 30 et 145°C, et encore mieux comprise entre 40 et 140°C, ou un mélange de tels monomères.

Ce ou ces monomères peuvent être choisis parmi les "monomères sélectionnés" ci-dessus mentionnés et/ou parmi les monomères additionnels.

Le ou les monomères de Tg $\geq$ 20°C peuvent donc être présents à raison de 1 à 99% en poids, notamment 10-90% en poids, encore mieux 20-80% en poids, voire 25-75% en poids, par rapport au poids total du copolymère.

A titre d'information, on notera que acrylate d'isobornyle a une Tg de 94°C, le méthacrylate d'isobornyle a une Tg de 110°C et le méthacrylate d'isobutyle a une Tg de 53°C, et sont donc dits de Tg haute, supérieure à 20°C, alors que l'acrylate d'isobutyle a une Tg de -24°C et l'acrylate d'éthyle-2-hexyle a une Tg de -50°C et sont donc dits de Tg basse, inférieure à 20°C.

Dans la présente description, on désignera par 'monomère de Tg' les monomères dont l'homopolymère a une telle Tg. Dans la présente invention, les Tg (ou température de transition vitreuse) sont des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$\frac{1}{Tg} = \sum_i \left(\frac{\varpi i}{Tgi}\right)$$

wi étant la fraction massique du monomère i dans la séquence considérée et Tgi étant la température de transition vitreuse de l'homopolymère du monomère i (en Kelvin).

L'homme du métier saura choisir les monomères et leurs quantités en fonction du résultat recherché, en se basant sur ses connaissances générales, notamment sur la réactivité relative de chaque monomère. En particulier, il choisira les monomères et leur quantité, ainsi que le milieu solvant, de manière à obtenir un copolymère préférentiellement soluble dans ledit milieu solvant.

Les copolymères à gradient de l'invention peuvent être préparés par l'homme du métier suivant le mode opératoire suivant :

1/ On prépare un mélange des différents monomères, éventuellement dans un solvant, de préférence dans un réacteur et sous agitation. On ajoute un initiateur de polymérisation radicalaire et un agent de contrôle de la polymérisation. Le mélange est mis de préférence sous atmosphère de gaz inerte par rapport à une polymérisation radicalaire tel que l'azote ou l'argon.

Comme solvant éventuel de polymérisation, on peut choisir le milieu solvant de la composition, qui peut donc comprendre les solvants et/ou les huiles cosmétiques tels que ci-après définis. En particulier, on peut choisir le solvant de polymérisation parmi les acétates d'alkyle tels que l'acétate de butyle ou l'acétate d'éthyle, des solvants aromatiques tels que le toluène ou des alcanes tels que l'isododécane, l'heptane ou l'isohexadécane.

2/ On porte le mélange sous agitation à la température de polymérisation souhaitée. Cette température est de préférence choisie dans une gamme allant de 10°C à 160°C, de préférence de 25°C à 130°C.

Le choix de la température de polymérisation est de préférence optimisé en fonction de la composition chimique du mélange de monomères. Ainsi, les monomères ayant des constantes cinétiques de propagation très élevée et une affinité pour l'agent de contrôle plus faible seront de préférence polymérisés à basse température.

3/ Le milieu de polymérisation est éventuellement modifié pendant la polymérisation, avant d'atteindre 90% de conversion des monomère initiaux, par ajout supplémentaire d'un ou plusieurs monomères, notamment du mélange initial. Cet ajout peut être réalisé de différentes manières, pouvant aller de l'addition brutale en une seule fois à l'addition continue sur la durée totale de la polymérisation.

4/ on arrête la polymérisation lorsque le taux de conversion souhaité est atteint. De cette conversion dépend la composition globale du copolymère. De préférence, on arrête la polymérisation après avoir atteint au moins 50% de conversion, notamment au moins 60%, préférentiellement après avoir atteint au moins 90% de conversion.

5/ Les éventuels monomères résiduels peuvent être éliminés par toute méthode connue, telle que par évaporation, ou par ajout d'une quantité d'initiateur classique de polymérisation tel que les dérivés peroxydiques ou azoïques.

Il est également possible d'effectuer une polymérisation en semi-continu, par exemple en introduisant dans un réacteur, tout ou partie du solvant éventuel, une partie des monomères, notamment 1 à 20% en poids du poids total de monomère, une partie de l'amorceur, notamment 1 à 20% en poids du poids total d'amorceur, puis en chauffant à la température requise. Le reste du solvant, des monomères et de l'amorceur peuvent être introduits par une coulée, en cours de polymérisation. Ils peuvent être introduits par des coulées distinctes ou identiques.

L'agent de contrôle de la polymérisation peut être (i) un nitroxyde ou une alcoxamine; (ii) un complexe organométallique en présence d'un composé halogéné; ou encore (iii) des dérivés soufrés de type xanthate, dithioester, trithiocarbonate ou carbamate.

L'initiateur de polymérisation radicalaire peut être choisi parmi tous les initiateurs de polymérisation usuels, tels que les composés de type azoïque et notamment l'azobisisobutyronitrile, ou de type peroxyde tels que les peroxydes organiques ayant 6-30 atomes de carbone, notamment le peroxyde de benzoyle.

L'initiateur de polymérisation radicalaire peut également être une alcoxyamine qui peut être avantageusement choisie pour initier la polymérisation et libérer en même temps le nitroxyde contrôlant cette polymérisation.

L'initiateur de polymérisation peut être un composé organique halogéné, notamment un alkyle ou ester halogéné, tel que l'éthyl-2-bromo-isobutyrate.

L'homme du métier saura choisir l'initiateur de polymérisation en fonction de la technique de polymérisation radicalaire contrôlée envisagée, et/ou en fonction des besoins de l'application. Ainsi, un initiateur mono-fonctionnel conduira à des chaînes dissymétriques, alors qu'un initiateur poly-fonctionnel conduira à des macromolécules ayant une symétrie à partir d'un coeur.

Les copolymères selon l'invention sont tout particulièrement solubles dans les milieux solvants lipophiles tels que les solvants notamment lipophiles et/ou les huiles carbonées, usuellement employés en cosmétique.

On rappelle que par "soluble", on entend que le polymère ne forme pas de précipité dans le solvant. Avantageusement,

le copolymère selon l'invention est soluble à une concentration d'au moins 1 % en poids, dans l'isododécane, à 25°C, 1 atm., de préférence à une concentration d'au moins 5% en poids, voire d'au moins 10% en poids.

Les copolymères à gradient selon l'invention peuvent être présents dans les compositions cosmétiques ou dermatologiques topiques en une quantité de 0,1 à 95% en poids, de préférence 0,5 à 90% en poids, notamment 1 à 80% en poids, voire 5-70% en poids, par rapport au poids total de la composition.

Les copolymères peuvent donc être présents dans la composition sous forme solubilisée, par exemple dans un solvant organique cosmétique ou une huile carbonée cosmétique.

On a constaté que les copolymères à gradients selon l'invention sont tout particulièrement solubles et qu'ils peuvent en outre être mis en solution en des quantités importantes sans influer sur la viscosité de la solution.

Les compositions cosmétiques ou dermatologiques selon l'invention comprennent, outre lesdits copolymères, un milieu physiologiquement acceptable, notamment cosmétiquement ou dermatologiquement acceptable, c'est-à-dire un milieu compatible avec les matières kératiniques telles que la peau du visage ou du corps, les lèvres, les cheveux, les cils, les sourcils et les ongles.

La composition peut avantageusement comprendre un milieux solvant qui peut être une phase grasse, qui peut elle-même comprendre des huiles et/ou des solvants de préférence lipophiles, ainsi que des corps gras solides à température ambiante tels que les cires, les corps gras pâteux, les gommes et leurs mélanges.

Parmi les constituants de la phase grasse, on peut préférentiellement citer les huiles et/ou solvants ayant un paramètre de solubilité global selon l'espace de solubilité de Hansen inférieur ou égal à 20 $(MPa)^{1/2}$, de préférence inférieur ou égal à 18 $(MPa)^{1/2}$, encore mieux inférieur ou égal à 17 $(MPa)^{1/2}$.

Le paramètre de solubilité global $\delta$ selon l'espace de solubilité de Hansen est défini dans l'article « Solubility parameter values » de Eric A.Grulke de l'ouvrage « Polymer Handbook », 3éme édition, Chapitre VII, p.519-559 par la relation :

$$\delta = (dD^2 + dP^2 + dH^2)^{1/2}$$

dans laquelle

- dD caractérise les forces de dispersion de LONDON issues de la formation de dipôles induits lors des chocs moléculaires,
- dP caractérise les forces d'interactions de DEBYE entre dipôles permanents, et
- dH caractérise les forces d'interactions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

Parmi les huiles et/ou solvants ayant un paramètre de solubilité global selon l'espace de solubilité de Hansen inférieur ou égal à 20 (MPa)1/2, on peut citer les huiles, volatiles ou non, qui peuvent être choisies parmi les huiles naturelles ou synthétiques, carbonées, hydrocarbonées, fluorées, éventuellement ramifiées, seules ou en mélange; les éthers et esters ayant plus de 6 atomes de carbone, notamment 6 à 30 atomes de carbone ; les cétones ayant plus de 6 atomes de carbone, notamment 6 à 30 atomes de carbone; les monoalcools gras aliphatiques ayant 6 à 30 atomes de carbone, la chaîne hydrocarbonée ne comportant pas de groupement de substitution.

On entend par "huile non volatile", une huile susceptible de rester sur la peau à température ambiante et pression atmosphérique au moins une heure et ayant notamment une pression de vapeur à température ambiante (25°C) et pression atmosphérique, non nulle, inférieure à 0,01 mm de Hg (1,33 Pa).

On peut en particulier citer les huiles non volatile carbonées, notamment hydrocarbonées, d'origine végétale, minérale, animale ou synthétique, telles que l'huile de paraffine (ou vaseline), le squalane, le polyisobutylène hydrogéné (huile de Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de

diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges.

On peut encore citer le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol.

On peut encore citer les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges.

Parmi les composés volatils, on peut citer les huiles volatiles non siliconées, notamment les isoparaffines en C8-C16 comme l'isododécane, l'isodécane, l'isohexadécane et par exemple les huiles vendues sous les noms commerciaux d'ISOPAR, de PERMETHYL et notamment l'isododécane (PERMETHYL 99 A).

Plus préférentiellement, on peut citer les alcanes liquides à température ambiante, volatils ou non, et plus particulièrement le décane, l'heptane, le dodécane, l'isododécane, l'isohexadécane, le cyclohexane, l'isodécane, et leurs mélanges.

Ces huiles et/ou solvants peuvent être généralement présents en une teneur allant de 0,01 à 95%, de préférence de 0,1 à 90%, de préférence encore de 10 à 85% en poids, par rapport au poids total de la composition, et mieux de 30 à 80%.

La composition peut en outre comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1 à 80% en poids, par rapport au poids total de la composition, et de préférence de 1 à 70% en poids.

La composition selon l'invention peut également comprendre des cires et/ou des gommes.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,01 à 50% en poids de cires, par rapport au poids total de la composition et mieux de 1 à 30 % en poids.

La composition selon l'invention peut en outre comprendre une ou des matières colorantes choisies parmi les colorants hydrosolubles, les colorants liposolubles, et les matières colorantes pulvérulentes comme les pigments, les nacres, et les paillettes bien connues de l'homme du métier. Les matières colorantes peuvent être présentes, dans la composition, en une teneur allant de 0,01 à 50% en poids, par rapport au poids de la composition, de préférence de 0,01 à 30% en poids.

Par pigments, il faut comprendre des particules de toute forme, blanches ou colorées, minérales ou organiques, insolubles dans le milieu physiologique, destinées à colorer la composition. Par nacres, il faut comprendre des particules de toute forme irisées, notamment produites par certains mollusques dans leur coquille ou bien synthétisées. Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium. Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition. Les charges peuvent être minérales ou organiques de toute forme, plaquettaires, sphériques ou oblongues, quelle que soit la forme cristallographique (par exemple feuillet, cubique, hexagonale, orthorhombique, etc.). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de

cisaillement de 200 $s^{-1}$, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).

La composition selon l'invention peut être une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel.

La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

Avantageusement, la composition selon l'invention peut être une composition de maquillage, notamment un fond de teint ou un rouge à lèvres.

L'invention a aussi pour objet un procédé cosmétique de maquillage ou de soin des matières kératiniques, notamment de la peau du corps ou du visage, des ongles, des cheveux et/ou des cils, comprenant l'application sur lesdites matières d'une composition cosmétique telle que définie précédemment.

En particulier, l'invention a pour objet un procédé cosmétique de maquillage de la peau du visage et/ou des lèvres, comprenant l'application sur lesdites matières d'une composition cosmétique de fond de teint ou de rouge à lèvres telles que définies précédemment.

## Exemple 1

Dans un tricol de 100 ml, équipé d'un réfrigérant et sous balayage d'argon, on mélange 3,5 g d'acrylate d'isobornyle ($1.7\ 10^{-2}$ mole), 3,5 g de methacrylate d'isobornyle ($1.6\ 10^{-2}$ mole), 3 g d'acrylate d'isobutyle ($2.3\ 10^{-2}$ mole) et 10 g d'acétate de butyle.

Ce mélange est dégazé par bullage d'argon pendant 15 minutes puis on ajoute 40 mg de CuBr, 49 mg de PMDETA (N, N,N',N',N"-pentamethyldiethylenetriamine) et 55,6 mg d'éthyl-2-bromoisobutyrate. Le mélange réactionnel est porté à 100°C pendant 2 heures puis filtré sur alumine.

On obtient une solution du polymère dans l'acétate de butyle (50% de matière sèche MS).

On peut distiller l'acétate de butyle et le substituer par de l'isododécane (10 g). On obtient alors une solution du polymère dans l'isododécane (50% MS).

## Exemple 2

Dans un tricol de 100 ml, équipé d'un réfrigérant et sous balayage d'argon, on mélange 3,5 g d'acrylate d'isobornyle, 3,5 g de methacrylate d'isobornyle, 3 g d'acrylate d'éthyle-2-hexyle et 10 g d'acétate de butyle.

Ce mélange est dégazé par bullage d'argon pendant 15 minutes puis on ajoute 40 mg de CuBr, 49 mg de PMDETA (N, N,N',N',N"-pentamethyldiethylenetriamine) et 55,6 mg d'éthyl-2-bromoisobutyrate. Le mélange réactionnel est porté à 100°C pendant 2 heures puis filtré sur alumine.

On obtient une solution du polymère dans l'acétate de butyle (50% de matière sèche MS).

On peut distiller l'acétate de butyle et le substituer par de l'isododécane (10 g). On obtient alors une solution du polymère dans l'isododécane (50% MS).

## Exemple 3

Dans un tricol de 100 ml, équipé d'un réfrigérant et sous balayage d'argon, on mélange 5 g d'acrylate d'isobornyle, 5 g d'acrylate d'isobutyle et 10 g d'acétate de butyle.

Ce mélange est dégazé par bullage d'argon pendant 15 minutes puis on ajoute 40 mg de CuBr, 49 mg de PMDETA (N, N,N',N',N"-pentamethyldiethylenetriamine) et 55,6 mg d'éthyl-2-bromoisobutyrate. Le mélange réactionnel est porté à

100°C pendant 2 heures puis filtré sur alumine.
On obtient une solution du polymère dans l'acétate de butyle (50% de matière sèche MS).
On peut distiller l'acétate de butyle et le substituer par de l'isododécane (10 g). On obtient alors une solution du polymère dans l'isododécane (50% MS).

**Exemple 4**

On prépare un vernis à ongles comprenant :

- 40% en poids de solution du polymère de l'exemple 1 à 50% MS dans l'acétate de butyle
- qsp 100% solvants organiques (acétate de butyle et acétate d'éthyle).

**Exemple 5**

On prépare un fond de teint anhydre comprenant (% en poids) :

- cire de polyéthylène 12%
- huiles siliconées volatiles 25%
- phényltriméthicone 20%
- Microsphères de polyméthylméthacrylate 12%
- Solution du polymère de l'exemple 2 à 50% MS dans l'isododécane (soit 6% de matière sèche de polymère) 12%
- Isododécane qsp 100%

Préparation :

On fait fondre les cires puis, quand tout est limpide, on ajoute la phényl triméthicone sous agitation, et les huiles de silicones; on ajoute ensuite les microsphères, l'isododécane et le polymère. On homogénéise pendant 15 minutes puis on coule la composition résultante que l'on laisse refroidir. On obtient un fond de teint anhydre.

**Exemple 6**

On prépare un stick de rouge à lèvres comprenant :

- Cire de polyéthylène 15%
- Solution du polymère de l'exemple 3 à 50% MS dans l'isododécane (soit 10% de matière sèche de polymère) 20%
- Polyisobutène hydrogéné (Parléam de Nippon Oil Fats) 25%
- Pigments 10%
- Isododécane qsp 100%

La composition obtenue après application sur les lèvres présente de bonnes propriétés cosmétiques.

**Exemple 7**

On prépare une composition de fond de teint comprenant (% en poids) :

Phase A

- Cetyl Dimethicone copolyol (ABIL EM 90 de GOLDSCHMIDT) 3 g
- Succinate d'isostéaryl diglycéryle (IMWITOR 780K de la société CONDEA) 0,6 g
- Pigments (oxydes de fer et de titane) 10 g
- Poudre de polyamide (NYLON-12) 8 g
- Solution du polymère de l'exemple 1 à 50% MS dans l'isododécane 17 g (soit 8,5% de matière sèche de polymère)
- Parfum qs
- Isododécane 10 g

Phase B

- Sulfate de magnésium 0,7 g
- Conservateur qs
- Eau qsp 100 g

La composition obtenue présente de bonnes propriétés cosmétiques.

## Revendications

1. Copolymère à gradient comprenant au moins deux monomères différents, choisis tous les deux dans la liste suivante : l'acrylate d'isobornyle, le méthacrylate d'isobornyle, l'acrylate d'isobutyle, le méthacrylate d'isobutyle et l'acrylate d'éthyle-2-hexyle.

2. Copolymère selon la revendication précédente, comprenant de 1 à 99% en poids par rapport au poids du copolymère final, notamment de 2-98% en poids, de préférence de 5-95% en poids, voire de 30 à 70% en poids, d'un premier monomère choisi dans la liste.

3. Copolymère selon l'une des revendications précédentes, comprenant de 1 à 99% en poids par rapport au poids du copolymère final, notamment de 2-98% en poids, de préférence de 5-95% en poids, voire de 30 à 70% en poids, d'un second monomère, différent du premier monomère, choisi dans la liste.

4. Copolymère selon l'une des revendications précédentes, comprenant en outre un troisième monomère choisi dans la liste, qui peut être présent de 1 à 50% en poids, notamment de 5 à 40% en poids, voire de 10 à 35% en poids, par rapport au poids du copolymère final.

5. Copolymère selon l'une des revendications précédentes, dans lequel la quantité totale de monomères choisis dans la liste, dans le copolymère final, est comprise entre 50 et 100% en poids inclus, notamment de 60 à 98% en poids, voire de 70 à 97% en poids, encore mieux de 80 à 96% en poids, préférentiellement de 90 à 95% en poids par rapport au poids du copolymère final.

6. Copolymère selon l'une des revendications précédentes, comprenant :

   - de l'acrylate d'isobornyle et du méthacrylate d'isobornyle;
   - de l'acrylate d'isobornyle et de l'acrylate d'isobutyle;
   - de l'acrylate d'isobornyle et du méthacrylate d'isobutyle;
   - de l'acrylate d'isobornyle et de l'acrylate d'éthyle-2-hexyle;
   - du méthacrylate d'isobornyle et de l'acrylate d'isobutyle;
   - du méthacrylate d'isobornyle et du méthacrylate d'isobutyle;
   - du méthacrylate d'isobornyle et de l'acrylate d'éthyle-2-hexyle;
   - de l'acrylate d'isobornyle, du méthacrylate d'isobornyle et de l'acrylate d'éthyle-2-hexyle;
   - de l'acrylate d'isobornyle, du méthacrylate d'isobornyle et de l'acrylate d'isobutyle;
   - de l'acrylate d'isobornyle, du méthacrylate d'isobutyle et de l'acrylate d'éthyle-2-hexyle;
   - de l'acrylate d'isobornyle, du méthacrylate d'isobutyle et de l'acrylate d'isobutyle; ou
   - du méthacrylate d'isobornyle, du méthacrylate d'isobutyle et de l'acrylate d'isobutyle.

7. Copolymère selon l'une des revendications précédentes, comprenant en outre au moins un monomère additionnel autre que ceux choisis dans la liste, qui peut être présent en une quantité de 0 à 50% en poids, notamment de 2 à 40% en poids, voire de 3 à 30% en poids, encore mieux de 4 à 20% en poids, préférentiellement de 5 à 10% en poids, par rapport au poids du copolymère final.

8. Copolymère selon la revendication 7, dans lequel le monomère additionnel est choisi parmi, seul ou en mélange, les monomères suivants, ainsi que leurs sels, à l'exclusion des monomères listés:

   - (i) les (méth)acrylates de formule $CH_2$=CHCOOR ou $CH_2$=C($CH_3$)COOR dans laquelle R représente :

     - un groupe alkyle linéaire ou ramifié, comprenant 1 à 30 atomes de carbone dans lequel se trouve(nt)

éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I et F), les groupes -NR4R5, où R4 et R5 identiques ou différents représentent l'hydrogène ou un groupe alkyle linéaire ou ramifié, en C1 à C6 ou un groupe phényle; et/ou les groupes polyoxyalkylènes, notamment polyoxyéthylène et/ou polyoxypropylène, ledit groupe polyoxyalkylène étant constitué par la répétition de 5 à 30 motifs oxyalkylène;

- un groupe cycloalkyle en C3 à C12, ledit groupe cycloalkyle pouvant comporter dans sa chaîne un ou plusieurs hétéroatomes choisis parmi O, N, S et/ou P, et/ou pouvant être éventuellement substitué par un ou plusieurs substituants choisis parmi -OH et les atomes d'halogène (Cl, Br, I et F);
- un groupe aryle en C4 à C20 ou aralkyle en C5 à C30 (groupe alkyle en C1 à C8);

notamment R peut être un groupe méthyle, éthyle, propyle, isopropyle, n-butyle, isobutyle, tertiobutyle, hexyle, éthylhexyle, octyle, lauryle, isooctyle, isodécyle, dodécyle, cyclohexyle, t-butylcyclohexyle, stéaryle, éthyl-2-perfluorohexyle, 2-hydroxyéthyle, 2-hydroxybutyle, 2-hydroxypropyle, méthoxyéthyle, éthoxyéthyle, méthoxy-propyle, isobornyle, phényle, 2-phényl-éthyle, t-butylbenzyle, benzyle, furfurylméthyle ou tétrahydrofurfurylméthyle, méthoxy-polyoxyéthylène (ou POE-méthyle); POE-béhényle, trifluoroéthyle; diméthylaminoéthyle, diéthylaminoéthyle, diméthylaminopropyle.

- (ii) les (méth)acrylamides de formule $CH_2$=CHCON$R_4R_5$ ou $CH_2$=C($CH_3$)CON$R_4R_5$
dans laquelle R4 et R5, identiques ou différents, représentent un atome d'hydrogène ou :

a) un groupe alkyle, linéaire ou ramifié, comportant de 1 à 18 atomes de carbone, dans lequel se trouve (nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R4R5), où R4 et R5 identiques ou différents représentent un groupe alkyle en C1 à C6 ou un groupe phényle ;
et notamment un groupe méthyle, éthyle, propyle, isopropyle, n-butyle, isobutyle, tertiobutyle, hexyle, isohexyle, cyclohexyle, éthylhexyle, octyle, isooctyle, décyle, isodécyle, cyclodécyle, dodécyle, cyclododécyle, isononyle, lauryle, t-butylcyclohexyle, stéaryle; éthyl-2-perfluorohexyle; ou un groupe hydroxyalkyle en C1-4 tel que 2-hydroxyéthyle, 2-hydroxybutyle et 2-hydroxypropyle; ou un groupe alcoxy(C1-4)-alkyle(C1-4) tel que méthoxyéthyle, éthoxyéthyle et méthoxy-propyle,
b) un groupe cycloalkyle en C3 à C12, tel que le groupe isobomyle, ou un groupe hétérocycloalkyle (alkyle de 1 à 4 atomes de carbone), tel que furfurylméthyle ou tétrahydrofurfurylméthyle,
c) un groupe aryle en C4 à C20 tel que le groupe phényle,
d) un groupe aralkyle en C5 à C30 (groupe alkyle en C1 à C8) tel que 2-phényl éthyle, t-butylbenzyle ou benzyle,

- (iii) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylamidopropanesulfonique; et les sels de ceux ci;
- (iv) les éthers de vinyle de formule $R_6$O-CH=$CH_2$ ou les esters de vinyle de formule : $R_6$-COO-CH=$CH_2$
dans laquelle $R_6$ représente un groupe alkyle linéaire ou ramifié, comprenant de 1 à 22 atomes, ou un groupe alkyle cyclique comportant de 3 à 6 atomes de carbone et/ou un groupe aromatique, par exemple de type benzénique, anthracénique, et naphtalénique ;
- (v) les composés vinyliques de formules: CH2=CH-R9, CH2=CH-CH2-R9 ou CH2=C(CH3)-CH2-R9
dans lesquelles R9 est un groupe hydroxyle, halogène (Cl ou F), NH2, OR10 où R10 représente un groupe phényle ou un groupe alkyle en C1 à C12 (le monomère est un éther de vinyle ou d'allyle); acétamide (NHCOCH3); un groupe OCOR11 où R11 représente un groupe alkyle de 2 à 12 carbones, linéaire ou ramifié (le monomère est un ester de vinyle ou d'allyle); ou un groupe choisi parmi:

- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I est F), et les groupes Si (R4R5), où R4 et R5 identiques ou différents représentent un groupe alkyle en C1 à C6 ou un groupe phényle ;
- un groupe cycloalkyle en C3 à C12 tel que isobornyle, cyclohexane,
- un groupe aryle en C3 à C20 tel que phényle,

- un groupe aralkyle en C4 à C30 (groupe alkyle en C1 à C8) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,

- (vi) le styrène et ses dérivés, notamment tels que le méthylstyrène, le chlorostyrène ou le chlorométhylstyrène;
- (vii) les monomères à insaturation éthylénique comprenant un ou plusieurs atomes de silicium tels que le méthacryloxypropyl triméthoxy silane, le méthacryloxypropyl tris(triméthylsiloxy)silane;
- ainsi que leurs sels et leurs mélanges.

**9.** Copolymère selon l'une des revendications 7 à 8, dans lequel le monomère additionnel est choisi parmi les (méth) acrylates de méthyle, de n-propyle, d'isopropyle, de n-butyle, de t-butyle, de cyclohexyle, de méthoxyéthyle, d'éthoxyéthyle, de trifluoroéthyle, de diméthylaminoéthyle, de diéthylaminoéthyle, de 2-hydroxypropyle, de 2-hydroxyéthyle; l'acide acrylique, l'acide méthacrylique, le (méth)acrylamide, le méthacryloxypropyl triméthoxy silane, le méthacryloxypropyl tris(triméthylsiloxy)silane; ainsi que leurs sels; et leurs mélanges.

**10.** Copolymère selon l'une des revendications 7 à 8, dans lequel le monomère additionnel est choisi parmi, seul ou en mélange les macromonomères carbonés ou siliconés ayant au moins un groupe terminal polymérisable, tels que, seuls ou en mélange, ainsi que leurs sels, :

- (i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate; les macromonomères de poly(acrylate de stéaryle) ou de poly (méthacrylate de stéaryle) à extrémité mono(méth)acrylate.
- (ii) les polyoléfines ayant un groupe terminal à insaturation éthylénique, en particulier ceux ayant un groupement terminal (méth)acrylate, et notamment les macromonomères suivants, étant entendu qu'ils ont un groupe terminal (méth)acrylate: les macromonomères de polyéthylène, les macromonomères de polypropylène, les macromonomères de copolymère polyéthylène/polypropylène, les macromonomères de copolymère polyéthylène/polybutylène, les macromonomères de polyisobutylène; les macromonomères de polybutadiène; les macromonomères de polyisoprène; les macromonomères de polybutadiène; les macromonomères de poly (éthylène/butylène)-polyisoprène.
- (iii) les polydiméthylsiloxanes à groupement terminal mono(méth)acrylate, et notamment ceux de formule (IIa) suivante :

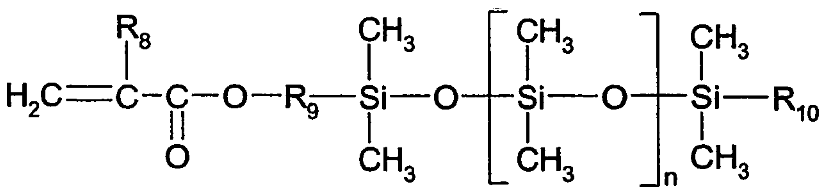

(IIa)

dans laquelle :

- $R_8$ désigne un atome d'hydrogène ou un groupement méthyle; de préférence méthyle;
- $R_9$ désigne un groupe hydrocarboné divalent, linéaire ou ramifié, de préférence linéaire, ayant de 1 à 10 atomes de carbone et contenant éventuellement une ou deux liaisons éther -O-; de préférence éthylène, propylène ou butylène;
- $R_{10}$ désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

**11.** Copolymère selon la revendication 10, dans lequel le monomère additionnel est choisi parmi les macromonomères monométhacryloyloxypropyl polydiméthylsiloxanes.

**12.** Copolymère selon l'une des revendications précédentes, présentant une masse moléculaire moyenne en poids comprise entre 2000 g/mol et 1 000 000 g/mol, notamment entre 3000 g/mol et 800 000 g/mol, et encore mieux entre 5000 g/mol et 500 000 g/mol.

**13.** Copolymère selon l'une des revendications précédentes, présentant une masse moléculaire moyenne en nombre comprise entre 2000 g/mol et 1 000 000 g/mol, notamment entre 3000 g/mol et 800 000 g/mol, et encore mieux entre 5000 g/mol et 500 000 g/mol.

**14.** Copolymère selon l'une des revendications précédentes, comprenant au moins un monomère ayant une Tg inférieure ou égale à 20°C, notamment comprise entre -150°C et 20°C, plus particulièrement comprise entre -130°C et 18°C, et encore mieux comprise entre -120°C et 15°C, ou un mélange de tels monomères.

**15.** Copolymère selon la revendication 14, dans lequel le ou les monomères de Tg ≤ 20°C sont présents à raison de 1 à 99% en poids, notamment 10-90% en poids, encore mieux 20-80% en poids, voire 25-75% en poids, par rapport au poids total du copolymère.

**16.** Copolymère selon l'une des revendications précédentes, comprenant au moins un monomère ayant une Tg supérieure ou égale à 20°C, notamment comprise entre 25 et 150°C, plus particulièrement comprise entre 30 et 145°C, et encore mieux comprise entre 40 et 140°C, ou un mélange de tels monomères.

**17.** Copolymère selon la revendication 16, dans lequel le ou les monomères de Tg ≥ 20°C sont présents à raison de 1 à 99% en poids, notamment 10-90% en poids, encore mieux 20-80% en poids, voire 25-75% en poids, par rapport au poids total du copolymère.

**18.** Copolymère selon l'une des revendications précédentes, **caractérisé par le fait qu'**il est soluble à une concentration d'au moins 1% en poids, dans l'isododécane, à 25°C, 1 atm., de préférence à une concentration d'au moins 5% en poids, voire d'au moins 10% en poids.

**19.** Copolymère selon l'une des revendications précédentes, présentant un indice de polydispersité en masse inférieur ou égal à 3, de préférence compris entre 1,1 et 2,5, notamment entre 1,15 et 2,3, voire entre 1,2 et 2,0, ou 1,9 ou encore 1,8

**20.** Copolymère selon l'une des revendications précédentes, présentant une différence ($V^{1/2}$ max - $V^{1/2}$ min) inférieure ou égale à 3,5, notamment comprise entre 1 et 2,8 et mieux entre 1,2 et 2,5; et/ou une valeur w comprise entre 1 et 3, notamment entre 1,1 et 2,3 et encore mieux entre 1,1 et 2,0.

**21.** Composition cosmétique ou dermatologique comprenant, dans un milieu physiologiquement acceptable, notamment cosmétiquement ou dermatologiquement acceptable, au moins un copolymère à gradient tel que défini selon l'une des revendications 1 à 20.

**22.** Composition selon la revendication 21, dans laquelle le copolymère est présent en une quantité de 0,1 à 95% en poids, de préférence 0,5 à 90% en poids, notamment 1 à 80% en poids, voire 5-70% en poids, par rapport au poids total de la composition.

**23.** Composition selon l'une des revendications 21 à 22, comprenant au moins une phase grasse, qui comprend des huiles et/ou des solvants ayant un paramètre de solubilité global selon l'espace de solubilité de Hansen inférieur ou égal à 20 $(MPa)^{1/2}$, de préférence inférieur ou égal à 18 $(MPa)^{1/2}$, encore mieux inférieur ou égal à 17 $(MPa)^{1/2}$.

**24.** Composition selon l'une des revendications 21 à 23, comprenant au moins une huile et/ou solvant choisi parmi les huiles, volatiles ou non, qui peuvent être choisies parmi les huiles naturelles ou synthétiques, carbonées, hydrocarbonées, fluorées, éventuellement ramifiées, seules ou en mélange; les éthers et esters ayant plus de 6 atomes de carbone, notamment 6 à 30 atomes de carbone ; les cétones ayant plus de 6 atomes de carbone, notamment 6 à 30 atomes de carbone; les monoalcools gras aliphatiques ayant 6 à 30 atomes de carbone, la chaîne hydrocarbonée ne comportant pas de groupement de substitution.

**25.** Composition selon l'une des revendications 21 à 24, comprenant au moins une huile et/ou solvant choisi parmi les huiles non volatile carbonées, notamment hydrocarbonées, d'origine végétale, minérale, animale ou synthétique, telles que l'huile de paraffine (ou vaseline), le squalane, le polyisobutylène hydrogéné (huile de Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools ayant de 6 à 20 atomes de carbone, notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges; le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol; les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges; les huiles volatiles non siliconées, notamment les isoparaffines en C8-C16 comme l'isododécane, l'isodécane, l'isohexadécane.

**26.** Composition selon l'une des revendications 21 à 25, comprenant au moins une huile et/ou solvant choisie parmi les alcanes liquides à température ambiante, volatils ou non, et plus particulièrement le décane, l'heptane, le dodécane, l'isododécane, l'isohexadécane, le cyclohexane, l'isodécane, et leurs mélanges.

**27.** Composition selon l'une des revendications 21 à 26, comprenant au moins de 0,01 à 95%, de préférence de 0,1 à 90%, de préférence encore de 10 à 85% en poids, par rapport au poids total de la composition, et mieux de 30 à 80%, d'au moins une huile et/ou solvant.

**28.** Composition selon l'une des revendications 21 à 27, comprenant en outre un constituant choisi parmi un milieu hydrophile comprenant de l'eau ou un mélange d'eau et de solvant organique hydrophile; les cires, les gommes; les matières colorantes; les charges; les polymères; les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges.

**29.** Composition selon l'une des revendications 21 à 28, se présentant sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan); sous forme anhydre, par exemple de pâte anhydre.

**30.** Composition selon l'une des revendications 21 à 29, se présentant sous forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux); d'une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel; d'une composition capillaire, notamment pour le maintien de la coiffure ou la mise en forme des cheveux.

**31.** Composition selon la revendication 30, se présentant sous forme d'une composition de maquillage, notamment un fond de teint ou un rouge à lèvres.

**32.** Procédé cosmétique de maquillage ou de soin des matières kératiniques, notamment de la peau du corps ou du visage, des ongles, des cheveux et/ou des cils, comprenant l'application sur lesdites matières d'une composition cosmétique telle que définie à l'une des revendications 21 à 31.

**33.** Procédé cosmétique de maquillage de la peau du visage et/ou des lèvres, comprenant l'application sur lesdites matières d'une composition cosmétique de fond de teint ou de rouge à lèvres telles que définies à la revendication 31.

## Claims

**1.** Gradient copolymer comprising at least two different monomers both chosen from the following list: isobornyl acrylate, isobornyl methacrylate, isobutyl acrylate, isobutyl methacrylate and 2-ethylhexyl acrylate.

**2.** Copolymer according to the preceding claim, comprising from 1 to 99% by weight, with respect to the weight of the final copolymer, in particular from 2 to 98% by weight, preferably from 5 to 95% by weight, indeed even from 30 to 70% by weight, of a first monomer chosen from the list.

**3.** Copolymer according to either of the preceding claims, comprising from 1 to 99% by weight, with respect to the weight of the final copolymer, in particular from 2 to 98% by weight, preferably from 5 to 95% by weight, indeed even from 30 to 70% by weight, of a second monomer, different from the first monomer, chosen from the list.

**4.** Copolymer according to one of the preceding claims, additionally comprising a third monomer chosen from the list which can be present from 1 to 50% by weight, in particular from 5 to 40% by weight, indeed even from 10 to 35% by weight, with respect to the weight of the final copolymer.

**5.** Copolymer according to one of the preceding claims, in which the total amount of monomers chosen from the list in the final copolymer is between 50 and 100% by weight inclusive, in particular from 60 to 98% by weight, indeed even from 70 to 97% by weight, better still from 80 to 96% by weight, preferably from 90 to 95% by weight, with respect to the weight of the final copolymer.

**6.** Copolymer according to one of the preceding claims, comprising:

- isobornyl acrylate and isobornyl methacrylate;
- isobornyl acrylate and isobutyl acrylate;
- isobornyl acrylate and isobutyl methacrylate;
- isobornyl acrylate and 2-ethylhexyl acrylate;
- isobornyl methacrylate and isobutyl acrylate;
- isobornyl methacrylate and isobutyl methacrylate;
- isobornyl methacrylate and 2-ethylhexyl acrylate;
- isobornyl acrylate, isobornyl methacrylate and 2-ethylhexyl acrylate;
- isobornyl acrylate, isobornyl methacrylate and isobutyl acrylate;
- isobornyl acrylate, isobutyl methacrylate and 2-ethylhexyl acrylate;
- isobornyl acrylate, isobutyl methacrylate and isobutyl acrylate; or
- isobornyl methacrylate, isobutyl methacrylate and isobutyl acrylate.

**7.** Copolymer according to one of the preceding claims, furthermore comprising at least one additional monomer, other than those chosen from the list, which can be present in an amount of 0 to 50% by weight, in particular of 2 to 40% by weight, indeed even of 3 to 30% by weight, better still of 4 to 20% by weight, preferably of 5 to 10% by weight, with respect to the weight of the final copolymer.

**8.** Copolymer according to Claim 7, in which the additional monomer is chosen, alone or as a mixture, from the following monomers and their salts, with the exception of the listed monomers:

- (i) (meth)acrylates of formula $CH_2$=CHCOOR or $CH_2$=C($CH_3$)COOR in which R represents:

  - a linear or branched alkyl group comprising 1 to 30 carbon atoms in which is (are) optionally inserted one or more heteroatoms chosen from O, N, S and P and/or said alkyl group being able to be optionally substituted by one or more substituents chosen from
  - OH, halogen atoms (Cl, Br, I and F), -$NR_4R_5$ groups, where $R_4$ and $R_5$, which are identical or different, represent hydrogen or a linear or branched $C_1$ to $C_6$ alkyl group or a phenyl group; and/or polyoxyalkylene groups, in particular polyoxyethylene and/or polyoxypropylene, said polyoxyalkylene group being composed of the repetition of 5 to 30 oxyalkylene units;
  - a $C_3$ to $C_{12}$ cycloalkyl group, said cycloalkyl group being able to comprise, in its chain, one or more heteroatoms chosen from O, N, S and/or P and/or being able to be optionally substituted by one or more substituents chosen from -OH and halogen atoms (Cl, Br, I and F);
  - a $C_4$ to $C_{20}$ aryl group or a $C_5$ to $C_{30}$ aralkyl group ($C_1$ to $C_8$ alkyl group);

in particular, R can be a methyl, ethyl, propyl, isopropyl, n-butyl, isobutyl, tert-butyl, hexyl, ethylhexyl, octyl, lauryl, isooctyl, isodecyl, dodecyl, cyclohexyl, t-butylcyclohexyl, stearyl, 2-ethyl-perfluorohexyl, 2-hydroxyethyl, 2-hydroxybutyl, 2-hydroxypropyl, methoxyethyl, ethoxyethyl, methoxypropyl, isobornyl, phenyl, 2-phenylethyl, t-butylbenzyl, benzyl, furfurylmethyl or tetrahydrofurfurylmethyl, methoxypolyoxyethylene (or POE-methyl), POE-behenyl, trifluoroethyl, dimethylaminoethyl, diethylaminoethyl or dimethylaminopropyl group,

- (ii) (meth)acrylamides of formula $CH_2$=$CHCONR_4R_5$ or $CH_2$=C($CH_3$)$CONR_4R_5$
in which $R_4$ and $R_5$, which are identical or different, represent a hydrogen atom or:

  a) a linear or branched alkyl group comprising from 1 to 18 carbon atoms in which is(are) optionally inserted one or more heteroatoms chosen from O, N, S and P; said alkyl group being able in addition to be optionally substituted by one or more substituents chosen from hydroxyl groups, halogen atoms (Cl, Br, I and F) and si($R_4R_5$) groups, where $R_4$ and $R_5$, which are identical or different, represent a $C_1$ to $C_6$ alkyl group or a phenyl group;
  and in particular a methyl, ethyl, propyl, isopropyl, n-butyl, isobutyl, tert-butyl, hexyl, isohexyl, cyclohexyl, ethylhexyl, octyl, isooctyl, decyl, isodecyl, cyclodecyl, dodecyl, cyclododecyl, isononyl, lauryl, t-butylcyclohexyl, stearyl or 2-ethylperfluorohexyl group; or a $C_{1-4}$ hydroxyalkyl group, such as 2-hydroxyethyl, 2-hydroxybutyl and 2-hydroxypropyl; or a ($C_{1-4}$)alkoxy($C_{1-4}$)alkyl group, such as methoxyethyl, ethoxyethyl and methoxypropyl,
  b) a $C_3$ to $C_{12}$ cycloalkyl group, such as the isobornyl group, or a heterocyclylalkyl group (alkyl of 1 to 4 carbon atoms), such as furfurylmethyl or tetrahydrofurfurylmethyl,
  c) a $C_4$ to $C_{20}$ aryl group, such as the phenyl group,
  d) a $C_5$ to $C_{30}$ aralkyl group ($C_1$ to $C_8$ alkyl group), such as 2-phenylethyl, t-butylbenzyl or benzyl,

- (iii) monomers possessing ethylenic unsaturation(s) comprising at least one carboxylic acid, phosphoric acid, sulphonic acid or anhydride functional group, such as, for example, acrylic acid, methacrylic acid, crotonic acid, maleic anhydride, itaconic acid, fumaric acid, maleic acid, styrenesulphonic acid, vinylbenzoic acid, vinylphosphonic acid, acrylamidopropanesulphonic acid and the salts of these;
- (iv) vinyl ethers of formula $R_6$O-CH=$CH_2$ or vinyl esters of formula $R_6$-COO-CH=$CH_2$
in which $R_6$ represents a linear or branched alkyl group comprising from 1 to 22 carbon atoms or a cyclic alkyl group comprising from 3 to 6 carbon atoms and/or an aromatic group, for example of benzene, anthracene and naphthalene type;
- (v) vinyl compounds of formula $CH_2$=CH-$R_9$, $CH_2$=CH=$CH_2$-$R_9$ or $CH_2$=C($CH_3$)-$CH_2$-$R_9$
in which $R_9$ is a hydroxyl group, a halogen group (Cl or F), an $NH_2$ group, an $OR_{10}$ group, where $R_{10}$ represents a phenyl group or a $C_1$ to $C_{12}$ alkyl group (the monomer is a vinyl or allyl ether), an acetamide group ($NHCOCH_3$), an $OCOR_{11}$ group, where $R_{11}$ represents a linear or branched alkyl group of 2 to 12 carbons (the monomer is a vinyl or allyl ester), or a group chosen from:

- a linear or branched alkyl group of 1 to 18 carbon atoms in which is(are) optionally inserted one or more heteroatoms chosen from 0, N, S and P, said alkyl group additionally being able to be optionally substituted by one or more substituents chosen from hydroxyl groups, halogen atoms (Cl, Br, I and F) and $Si(R_4R_5)$ groups, where $R_4$ and $R_5$, which are identical or different, represent a $C_1$ to $C_6$ alkyl group or a phenyl group;
- a $C_3$ to $C_{12}$ cycloalkyl group, such as isobornyl or cyclohexyl,
- a $C_3$ to $C_{20}$ aryl group, such as phenyl,
- a $C_4$ to $C_{30}$ aralkyl group ($C_1$ to $C_8$ alkyl group), such as 2-phenylethyl or benzyl,
- a 4- to 12-membered heterocyclic group comprising one or more heteroatoms chosen from O, N and S, the ring being aromatic or nonaromatic,
- a heterocyclylalkyl group (alkyl of 1 to 4 carbon atoms), such as furfurylmethyl or tetrahydrofurfurylmethyl,

- (vi) styrene and its derivatives, in particular such as methylstyrene, chlorostyrene or chloromethylstyrene;
- (vii) monomers possessing ethylenic unsaturation comprising one or more silicon atoms, such as methacryloyloxypropyltrimethoxysilane or methacryloyloxypropyltris(trimethylsiloxy)silane;
- and their salts and their mixtures.

**9.** Copolymer according to either of Claims 7 and 8, in which the additional monomer is chosen from methyl (meth) acrylates, n-propyl (meth)acrylates, isopropyl (meth)acrylates, n-butyl (meth)acrylates, t-butyl (meth)acrylates, cyclohexyl (meth)acrylates, methoxyethyl (meth)acrylates, ethoxyethyl (meth)acrylates, trifluoroethyl (meth)acrylates, dimethylaminoethyl (meth)acrylates, diethylaminoethyl (meth)acrylates, 2-hydroxypropyl (meth)acrylates and 2-hydroxyethyl (meth)acrylates, acrylic acid, methacrylic acid, (meth)acrylamide, methacryloyloxypropyltrimethoxysilane, methacryloyloxypropyltris-(trimethylsiloxy)silane, and their salts and their mixtures.

**10.** Copolymer according to either of Claims 7 and 8, in which the additional monomer is chosen, alone or as a mixture, from carbon-based or silicone macromonomers having at least one polymerizable end group, such as, alone or as a mixture, and their salts:

- (i) linear or branched $C_8$-$C_{22}$ alkyl (meth)acrylate homopolymers and copolymers exhibiting a polymerizable end group chosen from vinyl or (meth)acrylate groups, among which may be mentioned poly(2-ethylhexyl acrylate) macromonomers possessing a mono(meth)acrylate end; poly(dodecyl acrylate) or poly(dodecyl methacrylate) macromonomers possessing a mono(meth)-acrylate end; poly(stearyl acrylate) or poly(stearyl methacrylate) macromonomers possessing a mono(meth)-acrylate end;
- (ii) polyolefins having an end group possessing ethylenic unsaturation, in particular those having a (meth) acrylate end group and especially the following macromonomers, it being understood that they have a (meth) acrylate end group: polyethylene macromonomers, polypropylene macromonomers, polyethylene/polypropylene copolymer macromonomers, polyethylene/polybutylene copolymer macromonomers, polyisobutylene macromonomers, polybutadiene macromonomers, polyisoprene macromonomers, poly(ethylene/butylene)-polyisoprene macromonomers;
- (iii) polydimethylsiloxanes possessing a mono(meth)-acrylate end group, in particular those of following formula (IIa) :

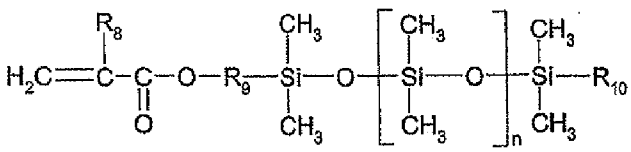

(IIa)

in which:

- $R_8$ denotes a hydrogen atom or a methyl group, preferably methyl;
- $R_9$ denotes a linear or branched, preferably linear, divalent hydrocarbon group having from 1 to 10 carbon atoms and optionally comprising one or two -0- ether bonds, preferably ethylene, propylene or butylene;
- $R_{10}$ denotes a linear or branched alkyl group having from 1 to 10 carbon atoms, in particular from 2 to 8 carbon atoms, preferably methyl, ethyl, propyl, butyl or pentyl;
- n denotes an integer ranging from 1 to 300, preferably ranging from 3 to 200 and preferentially ranging from 5 to 100.

11. Copolymer according to Claim 10, in which the additional monomer is chosen from monomethacryloyloxy-propylpolydimethylsiloxane macromonomers.

12. Copolymer according to one of the preceding claims, exhibiting a weight-average molecular weight of between 2000 g/mol and 1 000 000 g/mol, in particular between 3000 g/mol and 800 000 g/mol and better still between 5000 g/mol and 500 000 g/mol.

13. Copolymer according to one of the preceding claims, exhibiting a number-average molecular weight of between 2000 g/mol and 1 000 000 g/mol, in particular between 3000 g/mol and 800 000 g/mol and better still between 5000 g/mol and 500 000 g/mol.

14. Copolymer according to one of the preceding claims, comprising at least one monomer having a Tg of less than or equal to 20°C, in particular of between -150°C and 20°C, more particularly of between -130°C and 18°C and better still of between -120°C and 15°C, or a mixture of such monomers.

15. Copolymer according to Claim 14, in which the monomer or monomers with a $Tg \leq 20°C$ are present in a proportion of 1 to 99% by weight, in particular 10 to 90% by weight, better still 20 to 80% by weight, indeed even 25 to 75% by weight, with respect to the total weight of the copolymer.

16. Copolymer according to one of the preceding claims, comprising at least one monomer having a Tg of greater than or equal to 20°C, in particular of between 25 and 150°C, more particularly of between 30 and 145°C and better still of between 40 and 140°C, or a mixture of such monomers.

17. Copolymer according to Claim 16, in which the monomer or monomers with a $Tg \geq 20°C$ are present in a proportion of 1 to 99% by weight, in particular 10 to 90% by weight, better still 20 to 80% by weight, indeed even 25 to 75% by weight, with respect to the total weight of the copolymer.

18. Copolymer according to one of the preceding claims, **characterized in that** it is soluble at a concentration of at least 1% by weight in isododecane at 25°C, 1 atm., preferably at a concentration of at least 5% by weight, indeed even of at least 10% by weight.

19. Copolymer according to one of the preceding claims, exhibiting a weight polydispersity index of less than or equal to 3, preferably of between 1.1 and 2.5, in particular between 1.15 and 2.3, indeed even between 1.2 and 2.0, or 1.9 or even 1.8.

20. Copolymer according to one of the preceding claims, exhibiting a difference ($V^{1/2}$ max - $V^{1/2}$ min) of less than or equal to 3.5, in particular between 1 and 2.8 and better still between 1.2 and 2.5; and/or a value w of between 1 and 3, in particular between 1.1 and 2.3 and better still between 1.1 and 2.0.

21. Cosmetic or dermatological composition comprising, in a physiologically acceptable medium, in particular a cosmetically or dermatologically acceptable medium, at least one gradient copolymer as defined according to one of Claims 1 to 20.

22. Composition according to Claim 21, in which the copolymer is present in an amount of 0.1 to 95% by weight, preferably 0.5 to 90% by weight, in particular 1 to 80% by weight, indeed even 5 to 70% by weight, with respect to the total weight of the composition.

23. Composition according to either of Claims 21 and 22, comprising at least one fatty phase which comprises oils and/or solvents having an overall solubility parameter according to the Hansen solubility space of less than or equal to 20 $(MPa)^{1/2}$, preferably of less than or equal to 18 $(MPa)^{1/2}$, better still of less than or equal to 17 $(MPa)^{1/2}$.

24. Composition according to one of Claims 21 to 23, comprising at least one oil and/or solvent chosen from volatile or nonvolatile oils which can be chosen from optionally branched, carbon-based, hydrocarbon or fluorinated, natural or synthetic oils, alone or as a mixture; ethers and esters having more than 6 carbon atoms, in particular 6 to 30 carbon atoms; ketones having more than 6 carbon atoms, in particular 6 to 30 carbon atoms; aliphatic fatty monoalcohols having 6 to 30 carbon atoms, the hydrocarbon chain not comprising a substituent group.

25. Composition according to one of Claims 21 to 24, comprising at least one oil and/or solvent chosen from nonvolatile

carbon-based oils, in particular hydrocarbon oils, of vegetable, mineral, animal or synthetic origin, such as liquid paraffin (or liquid petrolatum), squalane, hydrogenated polyisobutylene (Parleam oil), perhydrosqualene, mink oil, macadamia nut oil, turtle oil, soybean oil, sweet almond oil, calophyllum oil, palm oil, grape seed oils, sesame oil, maize oil, arara oil, rapeseed oil, sunflower oil, cottonseed oil, apricot oil, castor oil, avocado oil, jojoba oil, olive oil, cereal germ oil or shea butter; linear, branched or cyclic esters having more than 6 carbon atoms, in particular 6 to 30 carbon atoms, such as esters of lanolic acid, of oleic acid, of lauric acid or of stearic acid; esters derived from acids or alcohols having from 6 to 20 carbon atoms, in particular esters of formula RCOOR' in which R represents the residue of a higher fatty acid comprising from 7 to 19 carbon atoms and R' represents a hydrocarbon chain comprising from 3 to 20 carbon atoms, in particular $C_{12}$-$C_{36}$ esters, such as isopropyl myristate, isopropyl palmitate, butyl stearate, hexyl laurate, diisopropyl adipate, isononyl isononanoate, 2-ethylhexyl palmitate, 2-hexyldecyl laurate, 2-octyldecyl palmitate, 2-octyldodecyl myristate or lactate, di(2-ethylhexyl) succinate, diisostearyl malate, glyceryl triisostearate or diglyceryl triisostearate; higher fatty acids, in particular $C_{14}$-$C_{22}$ fatty acids, such as myristic acid, palmitic acid, stearic acid, behenic acid, oleic acid, linoleic acid, linolenic acid or isostearic acid; higher fatty alcohols, in particular $C_{26}$-$C_{22}$ fatty alcohols, such as ketanol, oleyl alcohol, linoleyl alcohol, linolenyl alcohol, isostearyl alcohol, or octyldodecanol; and their mixtures; decanol, dodecanol, octadecanol, liquid triglycerides of fatty acids of 4 to 10 carbon atoms, such as triglycerides of heptanoic or octanoic acids or triglycerides of caprylic/capric acids; linear or branched hydrocarbons of mineral or synthetic origin, such as liquid paraffins and their derivatives, liquid petrolatum, polydecenes, hydrogenated polyisobutene, such as Parleam; synthetic esters and ethers, in particular of fatty acids, such as, for example, Purcellin oil, isopropyl myristate, 2-ethylhexyl palmitate, 2-octyldodecyl stearate, 2-octyldodecyl erucate or isostearyl isostearate; hydroxylated esters, such as isostearyl lactate, octyl hydroxystearate, octyldodecyl hydroxystearate, diisostearyl malate, triisocetyl citrate, heptanoates, octanoates, decanoates of fatty alcohols; polyol esters, such as propylene glycol dioctanoate, neopentyl glycol diheptanoate or diethylene glycol diisononanoate; pentaerythritol esters; fatty alcohols having from 12 to 26 carbon atoms, such as octyldodecanol, 2-butyloctanol, 2-hexyldecanol or 2-undecylpentadecanol; ketones which are liquid at ambient temperature, such as methyl ethyl ketone, methyl isobutyl ketone, diisobutyl ketone, isophorone, cyclohexanone or acetone; propylene glycol ethers which are liquid at ambient temperature, such as propylene glycol monomethyl ether, propylene glycol monomethyl ether acetate or dipropylene glycol mono(n-butyl) ether; short-chain esters (having a total of 3 to 8 carbon atoms), such as ethyl acetate, methyl acetate, propyl acetate, n-butyl acetate or isopentyl acetate; ethers which are liquid at ambient temperature, such as diethyl ether, dimethyl ether or dichlorodiethyl ether; alkanes which are liquid at ambient temperature, such as decane, heptane, dodecane, isododecane, isohexadecane or cyclohexane; aromatic cyclic compounds which are liquid at ambient temperature, such as toluene and xylene; aldehydes which are liquid at ambient temperature, such as benzaldehyde or acetaldehyde, and their mixtures; volatile non-silicone oils, in particular $C_8$-$C_{16}$ isoparaffins, such as isododecane, isodecane or isohexadecane.

**26.** Composition according to one of Claims 21 to 25, comprising at least one oil and/or solvent chosen from volatile or nonvolatile alkanes which are liquid at ambient temperature and more particularly decane, heptane, dodecane, isododecane, isohexadecane, cyclohexane, isodecane and their mixtures.

**27.** Composition according to one of Claims 21 to 26, comprising at least from 0.01 to 95%, preferably from 0.1 to 90%, more preferably from 10 to 85% by weight, with respect to the total weight of the composition, and better still from 30 to 80% of at least one oil and/or solvent.

**28.** Composition according to one of Claims 21 to 27, additionally comprising a constituent chosen from a hydrophilic medium comprising water or a mixture of water and of hydrophilic organic solvent; waxes or gums; colouring materials; fillers; polymers; vitamins, thickeners, gelling agents, trace elements, softening agents, sequestering agents, fragrances, basifying or acidifying agents, preservatives, sunscreens, surfactants, antioxidants, agents for combating hair loss, antidandruff agents, propellants, ceramides or their mixtures.

**29.** Composition according to one of Claims 21 to 28, which is provided in the form of a suspension, a dispersion, a solution, in particular organic, a gel, an emulsion, in particular oil-in-water (O/W) or water-in-oil (W/O) or multiple (W/O/W or polyol/O/W or O/W/O) emulsion, a cream, a foam, a dispersion of vesicles, in particular of ionic or nonionic lipids, a two-phase or multiphase lotion, a spray, a powder, a paste, in particular a soft paste (in particular a paste having a dynamic viscosity at 25°C of the order of 0.1 to 40 Pa·s under a shear rate of 200 $s^{-1}$, after measuring for 10 minutes by cone/plate geometry); in the anhydrous form, for example of an anhydrous paste.

**30.** Composition according to one of Claims 21 to 29, which is provided in the form of a makeup composition, in particular a product for the complexion, such as a foundation, a face powder or an eyeshadow; a product for the lips, such as a lipstick or a lip care product; a concealer; a blusher, a mascara or an eyeliner; a product for making up the eyebrows,

a lip pencil or an eye pencil; a product for the nails, such as a nail varnish or a nail care product; a product for making up the body; a product for making up the hair (hair mascara or lacquer); of a composition for protecting or caring for the skin of the face, of the neck, of the hands or of the body, in particular a composition for combating wrinkles or fatigue which makes it possible to give radiance to the skin, a moisturizing or treating composition; an antisun or artificial tanning composition; of a hair composition, in particular for the form retention of the hairstyle or the shaping of the hair.

**31.** Composition according to Claim 30, which is provided in the form of a makeup composition, in particular a foundation or a lipstick.

**32.** Cosmetic process for making up or caring for keratinous substances, in particular the skin of the body or face, the nails, the hair and/or the eyelashes, comprising the application, to said substances, of a cosmetic composition as defined according to one of Claims 21 to 31.

**33.** Cosmetic process for making up the skin of the face and/or lips, comprising the application, to said substances, of a foundation or lipstick cosmetic composition as defined according to Claim 31.

**Patentansprüche**

**1.** Gradientencopolymer, das mindestens zwei unterschiedliche Monomere enthält, die beide aus der folgenden Liste ausgewählt sind: Isobornylacrylat, Isobornylmethacrylat, Isobutylacrylat, Isobutylmethacrylat und 2-Ethylhexylacrylat.

**2.** Copolymer nach dem vorhergehenden Anspruch, das 1 bis 99 Gew.-%, insbesondere 2 bis 98 Gew.-%, vorzugsweise 5 bis 95 Gew.-% und besser 30 bis 70 Gew.-% eines ersten Monomers, das aus der Liste gewählt ist, bezogen auf das Gewicht des fertigen Copolymers, enthält.

**3.** Copolymer nach einem der vorhergehenden Ansprüche, das 1 bis 99 Gew.-%, insbesondere 2 bis 98 Gew.-%, vorzugsweise 5 bis 95 Gew.-% und besser 30 bis 70 Gew.-% eines zweiten Monomers, das von dem ersten Monomer verschieden ist und das aus der Liste gewählt ist, bezogen auf das Gewicht des fertigen Copolymers, enthält.

**4.** Copolymer nach einem der vorhergehenden Ansprüche, das ferner ein drittes Monomer enthält, das aus der Liste gewählt ist und das 1 bis 50 Gew.-%, insbesondere 5 bis 40 Gew.-% und besser 10 bis 35 Gew.-%, bezogen auf das Gewicht des fertigen Copolymers, ausmachen kann.

**5.** Copolymer nach einem der vorhergehenden Ansprüche, bei dem die Gesamtmenge der Monomere, die aus der Liste gewählt sind, in dem fertigen Copolymer im Bereich von 50 bis 100 Gew.-%, insbesondere 60 bis 98 Gew.-%, auch 70 bis 97 Gew.-%, noch besser 80 bis 96 Gew.-% und vorzugsweise 90 bis 95 Gew.-%, bezogen auf das Gewicht des fertigen Copolymers, liegt.

**6.** Copolymer nach einem der vorhergehenden Ansprüche, das enthält:

- Isobornylacrylat und Isobornylmethacrylat;
- Isobornylacrylat und Isobutylacrylat;
- Isobornylacrylat und Isobutylmethacrylat;
- Isobornylacrylat und 2-Ethylhexylacrylat;
- Isobornylmethacrylat und Isobutylacrylat;
- Isobornylmethacrylat und Isobutylmethacrylat;
- Isobornylmethacrylat und 2-Ethylhexylacrylat;
- Isobornylacrylat, Isobornylmethacrylat und 2-Ethylhexylacrylat;
- Isobornylacrylat, Isobornylmethacrylat und Isobutylacrylat;
- Isobornylacrylat, Isobutylmethacrylat und 2-Ethylhexylacrylat;
- Isobornylacrylat, Isobutylmethacrylat und Isobutylacrylat; oder
- Isobornylmethacrylat, Isobutylmethacrylat und Isobutylacrylat.

**7.** Copolymer nach einem der vorhergehenden Ansprüche, das ferner mindestens ein ergänzendes Monomer enthält, das von den aus der Liste ausgewählten Monomeren verschieden ist und das in einem Mengenanteil von 0 bis 50

Gew.-%, insbesondere 2 bis 40 Gew.-%, sogar 3 bis 30 Gew.-%, besser 4 bis 20 Gew.-% und vorzugsweise 5 bis 10 Gew.-%, bezogen auf das Gewicht des fertigen Copolymers, vorliegen kann.

**8.** Copolymer nach Anspruch 7, bei dem das ergänzende Monomer einzeln oder als Gemisch unter den folgenden Monomeren sowie deren Salzen mit Ausnahme der aufgelisteten Monomere ausgewählt ist:

- (i) (Meth)acrylaten der Formel der Formel $CH_2$=CHCOOR oder $CH_2$=C($CH_3$)COOR, wobei R bedeutet:

  - eine geradkettige oder verzweigte Alkylgruppe mit 1 bis 30 Kohlenstoffatomen, in die gegebenenfalls ein oder mehrere Heteroatome, die unter O, N, S und P ausgewählt sind, eingebaut sind und/oder wobei die Alkylgruppe gegebenenfalls mit einem oder mehreren Substituenten substituiert sein kann, die ausgewählt sind unter -OH, Halogenatomen (Cl, Br, I und F), den Gruppen -$NR_4R_5$, worin die Gruppen $R_4$ und $R_5$, die gleich oder verschieden sind, Wasserstoff oder eine geradkettige oder verzweigte $C_{1-6}$-Alkylgruppe oder eine Phenylgruppe bedeuten; und/oder Polyoxyalkylengruppen, insbesondere Polyethylenoxid und/ oder Polypropylenoxid, wobei die Polyoxyalkylengruppe aus 5 bis 30 Oxyalkylen-Wiederholungseinheiten gebildet ist;
  - eine Cycloalkylgruppe mit 3 bis 12 Kohlenstoffatomen, wobei die Cycloalkylgruppe in ihrer Kette ein oder mehrere Heteroatome enthalten kann, die unter O, N, S und/oder P ausgewählt sind, und/oder mit einem oder mehreren Substituenten substituiert sein kann, die ausgewählt sind unter -OH und Halogenatomen (Cl, Br, I und F);
  - eine Arylgruppe mit 4 bis 20 Kohlenstoffatomen oder eine Aralkylgruppe mit 5 bis 30 Kohlenstoffatomen ($C_{1-8}$-Alkylgruppe);

die Gruppe R kann insbesondere eine Gruppe Methyl, Ethyl, Propyl, Isopropyl, n-Butyl, Isobutyl, t-Butyl, Hexyl, Ethylhexyl, Octyl, Lauryl, Isooctyl, Isodecyl, Dodecyl, Cyclohexyl, t-Butylcyclohexyl, Stearyl, 2-Ethyl-perfluorhexyl, 2-Hydroxyethyl, 2-Hydroxybutyl, 2-Hydroxypropyl, Methoxyethyl, Ethoxyethyl, Methoxypropyl, Isobornyl, Phenyl, 2-Phenyl-ethyl, t-Butylbenzyl, Benzyl, Furfurylmethyl oder Tetrahydrofurfurylmethyl, Methoxypolyoxyethylen (oder PEO-methyl), PEO-behenyl, Trifluorethyl; Dimethylaminoethyl, Diethylaminoethyl, Dimethylaminopropyl sein,

- (ii) (Meth)acrylamiden der Formel $CH_2$=$CHCONR_4R_5$ oder $CH_2$=C($CH_3$)$CONR_4R_5$,

wobei $R_4$ und $R_5$, die gleich die verschieden sind, ein Wasserstoffatom bedeuten oder:

(a) eine geradkettige oder verzweigte Alkylgruppe, die 1 bis 18 Kohlenstoffatome enthält, in die gegebenenfalls ein oder mehrere Heteroatome, die unter O, N, S und P ausgewählt sind, eingebaut sind; wobei die Alkylgruppe gegebenenfalls ferner mit einem oder mehreren Substituenten substituiert sein kann, die ausgewählt sind unter Hydroxy, Halogenatomen (Cl, Br, I und F), den Gruppen Si($R_4R_5$), worin die Gruppen $R_4$ und $R_5$, die gleich oder verschieden sind, eine $C_{1-6}$-Alkylgruppe oder eine Phenylgruppe bedeuten; und insbesondere eine Gruppe Methyl, Ethyl, Propyl, Isopropyl, n-Butyl, Isobutyl, t-Butyl, Hexyl, Isohexyl, Cyclohexyl, Ethylhexyl, Octyl, Isooctyl, Decyl, Isodecyl, Cyclodecyl, Dodecyl, Cyclododecyl, Isononyl, Lauryl, t-Butylcyclohexyl, Stearyl; 2-Ethyl-perfluorhexyl; oder eine Hydroxyalkyl($C_{1-4}$)gruppe, beispielsweise 2-Hydroxyethyl, 2-Hydroxybutyl und 2-Hydroxypropyl; oder eine Alkoxy($C_{1-4}$)alkyl($C_{1-4}$)gruppe, wie Methoxyethyl, Ethoxyethyl oder Methoxypropyl,
(b) eine Cycloalkylgruppe mit 3 bis 12 Kohlenstoffatomen, wie Isobornyl, oder eine Heterocycloalkylgruppe (Alkylgruppe mit 1 bis 4 Kohlenstoffatomen), wie Furfurylmethyl oder Tetrahydrofurfurylmethyl,
c) eine Arylgruppe mit 4 bis 20 Kohlenstoffatomen, wie Phenyl,
d) eine Aralkylgruppe mit 5 bis 30 Kohlenstoffatomen (Alkylgruppe mit 1 bis 8 Kohlenstoffatomen), wie 2-Phenyl-ethyl, t-Butylbenzyl oder Benzyl,

- (iii) Monomeren mit einer oder mehreren ethylenisch ungesättigten Bindung(en), die mindestens eine Carbonsäurefunktion, Phosphorsäurefunktion oder Sulfonsäurefunktion oder Anhydridfunktion enthalten, wie beispielsweise Acrylsäure, Methacrylsäure, Crotonsäure, Maleinsäureanhydrid, Itaconsäure, Fumarsäure, Maleinsäure, Styrolsulfonsäure, Vinylbenzoesäure, Vinylphosphorsäure, Acrylamidopropansulfonsäure; und den Salzen dieser Verbindungen;
- (iv) Vinylethern der Formel $R_6$O-CH=$CH_2$ oder Vinylestern der Formel $R_6$COO-CR=$CH_2$,

worin $R_6$ eine geradkettige oder verzweigte Alkylgruppe mit 1 bis 22 Kohlenstoffatomen oder eine cyclische Alkylgruppe mit 3 bis 6 Kohlenstoffatomen und/ oder eine aromatische Gruppe bedeutet, beispielsweise vom Benzoltyp, Anthracentyp und Naphthalintyp;

- (v) Vinylverbindungen der Formeln $CH_2$=CH-$R_9$, $CH_2$=CH-$CH_2$-$R_9$ oder $CH_2$=C($CH_3$)-$CH_2$-$R_9$,

worin $R_9$ eine Hydroxygruppe, Halogen (Cl oder F), $NH_2$, $OR_{10}$, wobei $R_{10}$ eine Phenylgruppe oder $C_{1-12}$-Alkylgruppe bedeutet (das Monomer ist ein Vinylether oder Allylether); Acetamid ($NHCOCH_3$); eine Gruppe $OCOR_{11}$, worin $R_{11}$ eine geradkettige oder verzweigte Alkylgruppe mit 2 bis 12 Kohlenstoffatomen ist (das Monomer ist ein Vinylester oder Allylester); oder eine Gruppe bedeutet, die ausgewählt ist unter:

- einer geradkettigen oder verzweigten Alkylgruppe mit 1 bis 18 Kohlenstoffatomen, worin gegebenenfalls in der Kette ein oder mehrere Heteroatome enthalten ist (sind), die unter O, N, S und P ausgewählt sind; wobei die Alkylgruppe ferner gegebenenfalls mit einem oder mehreren Substituenten substituiert sein kann, die unter den Hydroxygruppen, Halogenatomen (Cl, Br, I und F) und Gruppen $Si(R_4R_5)$ ausgewählt sind, wobei $R_4$ und $R_5$, die gleich oder verschieden sind, eine $C_{1-6}$-Alkylgruppe oder eine Phenylgruppe bedeuten;
- einer Cycloalkylgruppe mit 3 bis 12 Kohlenstoffatomen, wie Isobornyl, Cyclohexan,
- einer Arylgruppe mit 3 bis 20 Kohlenstoffatomen, wie Phenyl,
- einer Aralkylgruppe mit 4 bis 30 Kohlenstoffatomen (Alkylgruppe mit C1 - C8), wie 2-Phenylethyl; Benzyl,
- einer heterocyclischen Gruppe mit 4 bis 12 Bestandteilen, die ein oder mehrere Heteroatome enthält, die unter O, N und S ausgewählt sind, wobei der Ring aromatisch oder nichtaromatisch sein kann,
- einer Heterocycloalkylgruppe ($C_{1-4}$-Alkyl), wie Furfurylmethyl oder Tetrahydrofurfurylmethyl,

- (vi) Styrol und seinen Derivaten, insbesondere Methylstyrol, Chlorstyrol oder Chlormethylstyrol;
- (vii) Monomeren mit ethylenisch ungesättigter Bindung, die ein oder mehrere Siliciumatome enthalten, wie Methacryloxypropyltrimethoxysilan, Methacryloxypropyltris(trimethylsiloxy)silan;
- sowie ihren Salzen und ihren Gemischen.

**9.** Copolymer nach einem der Ansprüche 7 bis 8, wobei das ergänzende Monomer unter Methyl(meth)acrylat, *n*-Propyl (meth)-acrylat, Isopropyl(meth)acrylat, *n*-Butyl(meth)acrylat, *t*-Butyl-(meth)acrylat, Cyclohexyl(meth)acrylat, Methoxyethyl(meth)-acrylat, Ethoxyethyl(meth)acrylat, Trifluorethyl(meth)acrylat, Dimethylaminoethyl(meth)acrylat, Diethylaminoethyl(meth)-acrylat, 2-Hydroxypropyl(meth)acrylat, 2-Hydroxyethyl(meth)-acrylat; Acrylsäure, Methacrylsäure, (Meth)acrylamid, Methacryloxypropyltrimethoxysilan, Methacryloxypropyltris(trimethylsiloxy)silan; sowie ihren Salzen; und ihren Gemischen ausgewählt ist.

**10.** Copolymer nach einem der Ansprüche 7 bis 8, wobei das ergänzende Monomer, einzeln oder als Gemisch, unter den Kohlenstoffmakromonomeren oder Siliciummakromonomeren ausgewählt ist, die mindestens eine polymerisierbare endständige Gruppe aufweisen, wie beispielsweise, einzeln oder als Gemisch, den folgenden Verbindungen sowie deren Salzen:

- (i) Homopolymeren und Copolymeren von Alkyl(meth)acrylat mit linearer oder verzweigter $C_{8-22}$-Alkylgruppe, die eine endständige polymerisierbare Gruppe aufweisen, die unter Vinyl oder (Meth)acrylat ausgewählt ist, wobei von diesen die Makromonomere von Poly(2-ethylhexylacrylat) mit Mono(meth)acrylat-Endgruppe; Makromonomere von Poly(dodecylacrylat) oder Poly(dodecylmethacrylat) mit Mono(meth)acrylat-Endgruppe; die Makromonomere von Poly(stearylacrylat) oder Poly(stearylmethacrylat) mit Mono(meth)acrylat-Endgruppe angegeben werden können.
- (ii) Polyolefinen, die eine endständige Gruppe mit ethylenisch ungesättigter Bindung aufweisen, insbesondere solchen mit einer endständigen (Meth)acrylatgruppe; und besonders den folgenden Makromonomeren, mit der Maßgabe, dass sie eine endständige (Meth)acrylatgruppe aufweisen: Makromonomeren von Polyethylen, Makromonomeren von Polypropylen, Makromonomeren aus Polyethylen/Polypropylen-Copolymer, Makromonomeren aus Polyethylen/Polybutylen-Copolymer, Makromonomeren von Polyisobutylen; Makromonomeren von Polybutadien; Makromonomeren von Polyisopren; Makromonomeren von Polybutadien; Makromonomeren von Poly(ethylen/butylen)-polyisopren;
- (iii) Polydimethylsiloxanen mit endständiger Mono(meth)-acrylatgruppe und besonders solchen der folgenden Formel (IIa):

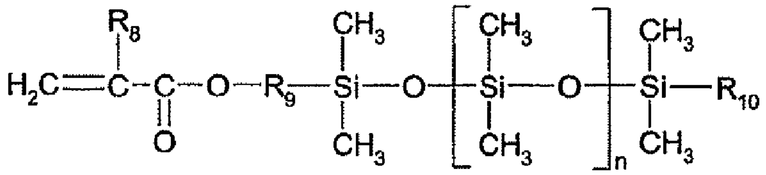

(IIa)

worin bedeuten:

- $R_8$ ein Wasserstoffatom oder die Methylgruppe; vorzugsweise Methyl;
- $R_9$ eine zweiwertige, geradkettige oder verzweigte und vorzugsweise lineare Kohlenwasserstoffgruppe mit 1 bis 10 Kohlenstoffatomen, die gegebenenfalls eine oder zwei Etherbindungen -O-aufweist; vorzugsweise Ethylen, Propylen oder Butylen;
- $R_{10}$ eine geradkettige oder verzweigte Alkylgruppe mit 1 bis 10 Kohlenstoffatomen, insbesondere 2 bis 8 Kohlenstoffatomen; bevorzugt Methyl, Ethyl, Propyl, Butyl oder Pentyl;
- n eine ganze Zahl von 1 bis 300, vorzugsweise 3 bis 200 und bevorzugt 5 bis 100.

**11.** Copolymer nach Anspruch 10, wobei das ergänzende Monomer unter den Monomethacryloyloxypropylpolydimethylsiloxan-Makromonomeren ausgewählt ist.

**12.** Copolymer nach einem der vorhergehenden Ansprüche, das eine gewichtsmittlere Molmasse von 2 000 bis 1 000 000 g/mol, insbesondere 3 000 bis 800 000 g/mol und noch besser 5 000 bis 500 000 g/mol aufweist.

**13.** Copolymer nach einem der vorhergehenden Ansprüche, das eine zahlenmittlere Molmasse von 2 000 bis 1 000 000 g/mol, insbesondere 3 000 bis 800 000 g/mol und besser 5 000 bis 500 000 g/mol aufweist.

**14.** Copolymer nach einem der vorhergehenden Ansprüche, das mindestens ein Monomer, das eine Tg kleiner oder gleich 20 °C, insbesondere im Bereich von -150 bis 20 °C, besonders bevorzugt im Bereich von -130 bis 18 °C und besser im Bereich von -120 bis 15 °C aufweist, oder ein Gemisch solcher Monomere umfasst.

**15.** Copolymer nach Anspruch 14, wobei das oder die Monomere mit einer Tg ≤ 20 °C in einen Mengenanteil von 1 bis 99 Gew.-%, insbesondere 10 bis 90 Gew.-%, besser 20 bis 80 Gew.-% und noch besser 25 bis 75 Gew.-%, bezogen auf das Gesamtgewicht des Copolymers, enthalten sind.

**16.** Copolymer nach einem der vorhergehenden Ansprüche, das mindestens ein Monomer, das eine Tg von mindestens 20 °C, insbesondere im Bereich von 25 bis 150 °C, besonders im Bereich von 30 bis 145 °C und noch besser im Bereich von 40 bis 140 °C aufweist, oder ein Gemisch solcher Monomere enthält.

**17.** Copolymer nach Anspruch 16, wobei das oder die Monomere mit einer Tg ≥ 20 °C in einen Mengenanteil von 1 bis 99 Gew.-%, insbesondere 10 bis 90 Gew.-%, besser 20 bis 80 Gew.-% und noch besser 25 bis 75 Gew.-%, bezogen auf das Gesamtgewicht des Copolymers, enthalten sind.

**18.** Copolymer nach einem der vorhergehenden Ansprüche, **dadurch gekennzeichnet, dass** es bei 25 °C und 1 atm in einer Konzentration von mindestens 1 Gew.-% in Isododecan löslich ist, vorzugsweise in einer Konzentration von mindestens 5 Gew.-% und besser mindestens 10 Gew.-%.

**19.** Copolymer nach einem der vorhergehenden Ansprüche, das eine auf die Masse bezogene Polydispersität kleiner oder gleich 3, vorzugsweise im Bereich von 1,1 bis 2,5, insbesondere im Bereich von 1,15 bis 2,3, sogar im Bereich von 1,2 bis 2,0 oder von 1,9 oder von 1,8 aufweist.

**20.** Copolymer nach einem der vorhergehenden Ansprüche, das einen Unterschied ($V^{1/2}$max-$V^{1/2}$min) von kleiner oder gleich 3,5, insbesondere im Bereich von 1 bis 2,8, besser 1,2 bis 2,5 und/oder einen Wert w im Bereich von 1 bis 3, insbesondere 1,1 bis 2,3 und besser 1,1 bis 2,0 aufweist.

**21.** Kosmetische oder dermatologische Zusammensetzung, die in einem physiologisch akzeptablen Medium und insbesondere einem kosmetisch oder dermatologisch akzeptablen Medium mindestens ein Gradientencopolymer, wie es in einem der Ansprüche 1 bis 20 definiert ist, enthält.

**22.** Zusammensetzung nach Anspruch 21, wobei das Copolymer in einen Mengenanteil von 0,1 bis 95 Gew.-%, vorzugsweise 0,5 bis 90 Gew.-%, insbesondere 1 bis 80 Gew.-% und sogar 5 bis 70 Gew.-%, bezogen auf das Gesamtgewicht der Zusammensetzung, vorliegt.

**23.** Zusammensetzung nach einem der Ansprüche 21 bis 22, die mindestens eine Fettphase enthält, die Öle und/oder Lösemittel mit einem Gesamtsolubilitätsparameter im Solubilitätsraum nach Hansen von 20 $(MPa)^{1/2}$ oder darunter, vorzugsweise höchstens 18 $(MPa)^{1/2}$ und besser höchstens 17 $(MPa)^{1/2}$ umfasst.

**24.** Zusammensetzung nach einem der Ansprüche 21 bis 23, die mindestens ein Öl und/oder Lösemittel enthält, die ausgewählt sind unter den flüchtigen oder nicht flüchtigen Ölen, die unter den natürlichen oder synthetischen Ölen auf Kohlenstoffbasis, Kohlenwasserstoffölen, fluorierten Ölen, die gegebenenfalls verzweigt sind, einzeln oder in Form von ihren Gemischen ausgewählt sein können; Ethern und Estern mit mehr als 6 Kohlenstoffatomen und insbesondere 6 bis 30 Kohlenstoffatomen; Ketonen mit mehr als 6 Kohlenstoffatomen, insbesondere 6 bis 30 Kohlenstoffatomen; aliphatischen Monofettalkoholen mit 6 bis 30 Kohlenstoffatomen, wobei die Kohlenwasserstoffkette keine Substituentengruppe aufweist.

**25.** Zusammensetzung nach einem der Ansprüche 21 bis 243, die mindestens ein Öl und/oder Lösemittel enthält, die ausgewählt sind unter: nichtflüchtigen Ölen auf Kohlenstoffbasis und insbesondere Kohlenwasserstoffölen pflanzlicher, mineralischer, tierischer oder synthetischer Herkunft, wie Paraffinöl (oder Vaseline), Squalan, hydriertem Polyisobutylen (Parleamöl), Perhydrosqualen, Nerzöl, Macadamiaöl, Schildkrötenöl, Sojaöl, Süßmandelöl, Calophyllumöl, Palmöl, Traubenkernöl, Sesamöl, Maisöl, Araraöl, Rapsöl, Sonnenblumenöl, Baumwollsamenöl, Aprikosenkernöl, Ricinusöl, Avocadoöl, Jojobaöl, Olivenöl oder Getreidekeimölen, Sheabutter; linearen, verzweigten oder cyclischen Estern mit mehr als 6 Kohlenstoffatomen, insbesondere 6 bis 30 Kohlenstoffatomen, wie Estern von Lanolinsäure, Ölsäure, Laurinsäure, Stearinsäure; Estern, die von Säuren oder Alkoholen mit 6 bis 20 Kohlenstoffatomen abgeleitet sind, insbesondere Estern der Formel RCOOR', wobei R den Rest einer höheren Fettsäure mit 7 bis 19 Kohlenstoffatomen und R' eine Kohlenwasserstoffkette mit 3 bis 20 Kohlenstoffatomen bedeutet, insbesondere $C_{12-36}$-Estern, wie Isopropylmyristat, Isopropylpalmitat, Butylstearat, Hexyllaurat, Diisopropyladipat, Isononylisononanoat, 2-Ethylhexylpalmitat, 2-Hexyldecyllaurat, 2-Octyldecylpalmitat, 2-Octyldodecylmyristat, 2-Octyldodecyllactat, Di(2-Ethylhexyl)succinat, Diisostearlymalat, Glyceryltriisostearat oder Diglyceryltriisostearat; höheren Fettsäuren, insbesondere mit 14 bis 22 Kohlenstoffatomen, wie Myristinsäure, Palmitinsäure, Stearinsäure, Behensäure, Ölsäure, Linolsäure, Linolensäure oder Isostearinsäure; höheren Fettalkoholen, insbesondere mit 16 bis 22 Kohlenstoffatomen, wie Cetanol, Oleylalkohol, Linoleylalkohol oder Linolenylalkohol, Isostearylalkohol oder Octyldodecanol; und deren Gemischen; Decanol, Dodecanol, Octadecanol, flüssigen Triglyceriden von Fettsäuren mit 4 bis 10 Kohlenstoffatomen, wie Triglyceriden von Heptansäure oder Octansäure, Triglyceriden von Capryl/ caprinsäure; linearen oder verzweigten Kohlenwasserstoffen mineralischer oder synthetischer Herkunft, wie Paraffinölen und deren Derivaten, Vaseline, Polydecenen, hydriertem Polyisobuten, wie Parleam; synthetischen Estern und Ethern, insbesondere von Fettsäuren, wie beispielsweise Purcellinöl, Isopropylmyristat, 2-Ethylhexylpalmitat, 2-Octyldodecylstearat, 2-Octyldodecylerucat, Isostearylisostearat; hydroxylierten Estern, wie Isostearyllactat, Octylhydroxystearat, Octyldodecylhydroxystearat, Diisostearylmalat, Triisocetylcitrat, Heptanoaten, Octanoaten und Decanoaten von Fettalkoholen; Polyolestern, wie Propylenglycoldioctanoat, Neopentylglycoldiheptanoat, Diethylenglycoldiisononanoat; und Estern von Pentaerythrit; Fettalkoholen mit 12 bis 26 Kohlenstoffatomen, wie Octyldodecanol, 2-Butyloctanol, 2-Hexyldecanol, 2-Undecylpentadecanol; bei Raumtemperatur flüssigen Ketonen, wie Methylethylketon, Methylisobutylketon, Diisobutylketon, Isophoron, Cyclohexanon, Aceton; bei Raumtemperatur flüssigen Propylenglycolethern, wie Propylenglycolmonomethylether, Propylenglycolmonomethyletheracetat, Dipropylenglycolmono-*n*-butylether; kurzkettigen Estern (mit 3 bis 8 Kohlenstoffatomen insgesamt), wie Ethylacetat, Methylacetat, Propylacetat, *n*-Butylacetat, Isopentylacetat; bei Raumtemperatur flüssigen Ethern, wie Diethylether, Dimethylether oder Dichlordiethylether; bei Raumtemperatur flüssigen Alkanen, wie Decan, Heptan, Dodecan, Isododecan, Isohexadecan, Cyclohexan; bei Raumtemperatur flüssigen aromatischen cyclischen Verbindungen, wie Toluol und Xylol; bei Raumtemperatur flüssigen Aldehyden, wie Benzaldehyd, Acetaldehyd und deren Gemischen; nichtsiliconierten flüchtigen Ölen, insbesondere $C_{8-16}$-Isoparaffinen, wie Isododecan, Isodecan und Isohexadecan.

**26.** Zusammensetzung nach einem der Ansprüche 21 bis 25, die mindestens ein Öl und/oder Lösemittel enthält, die unter den bei Raumtemperatur flüssigen, flüchtigen oder nichtflüchtigen Alkanen und insbesondere Decan, Heptan, Dodecan, Isododecan, Isohexadecan, Cyclohexan, Isodecan und deren Gemischen ausgewählt sind.

**27.** Zusammensetzung nach einem der Ansprüche 21 bis 26, die mindestens 0,01 bis 95 %, vorzugsweise 0,1 bis 90 % und noch bevorzugter 10 bis 85 Gew.-%, bezogen auf das Gesamtgewicht der Zusammensetzung, und besser 30 bis 80 % mindestens eines Öls und/oder Lösungsmittels enthält.

**28.** Zusammensetzung nach einem der Ansprüche 21 bis 27, die ferner einen Bestandteil enthält, der ausgewählt ist unter: einem hydrophilen Medium, das Wasser oder ein Gemisch von Wasser und einem hydrophilen organischen Lösungsmittel enthält; Wachsen, Gummis; Farbmitteln; Füllstoffe; Polymeren; Vitaminen, Verdickungsmitteln, Gelbildnern, Spurenelementen, beruhigenden Stoffen, Maskierungsmitteln, Parfums, Alkalisierungsmitteln oder Ansäuerungsmitteln, Konservierungsmitteln, Sonnenschutzfiltern, grenzflächenaktiven Stoffen, Antioxidantien, Wirkstoffen gegen Haarausfall, Antischuppenmitteln, Treibmitteln, Ceramiden oder deren Gemischen.

**29.** Zusammensetzung nach einem der Ansprüche 21 bis 28, die in Form einer Suspension, Dispersion, Lösung und besonders organischen Lösung, als Gel, Emulsion und insbesondere Öl-in-Wasser-Emulsion (O/W) oder Wasser-in-Öl-Emulsion (W/O) oder multiple Emulsion (W/O/W oder Polyol/O/W oder O/W/O), in Form einer Creme, Paste, Mousse, Vesikeldispersion, insbesondere von ionischen oder nichtionischen Lipiden, als zweiphasige oder mehrphasige Lotion, Spray, Pulver, Paste und insbesondere weiche Paste (besonders als Paste mit einer dynamischen Viskosität in der Größenordnung von 0,1 bis 40 Pa.s bei 25 °C und einer Schergeschwindigkeit von 200 $s^{-1}$ nach 10-minütiger Messung in Kegel/Platte-Geometrie); in wasserfreier Form, beispielsweise als wasserfreie Paste, vorliegt.

**30.** Zusammensetzung nach einem der Ansprüche 21 bis 29, die in Form einer Zusammensetzung zum Schminken vorliegt, insbesondere als Produkt für den Teint, wie als Make-up, Wangenrouge oder Lidschatten; Produkt für die Lippen, wie als Lippenstift oder Lippenpflege; Produkt gegen Augenringe, Blush, Mascara, Eyeliner; Produkt zum Schminken der Augenbrauen, Lippencrayon oder Lidstrichstift; Produkt für die Nägel, wie als Nagellack oder Nagelpflegeprodukt; Produkt zum Schminken des Körpers; Produkt zum Schminken der Haare (Mascara oder Lack für die Haare); Zusammensetzung zum Schutz oder für die Pflege des Gesichts, des Halses, der Hände oder des Körpers und insbesondere als Zusammensetzung gegen Falten und gegen Müdigkeit, das den Teint der Haut zum Strahlen bringen kann, hydratisierende Zusammensetzung oder Zusammensetzung für die Behandlung; Sonnenschutzzusammensetzung oder Zusammensetzung für die künstliche Bräunung; Zusammensetzung für die Haarbehandlung und insbesondere für die Festigung oder Formgebung der Haare vorliegt.

**31.** Zusammensetzung nach Anspruch 30, die in Form einer Zusammensetzung zum Schminken vorliegt, besonders als Make-up oder Lippenstift.

**32.** Kosmetisches Verfahren zum Schminken oder für die Pflege von Keratinsubstanzen und insbesondere der Haut des Körpers oder des Gesichts, der Nägel, der Haare und/oder der Wimpern, das das Auftragen einer kosmetischen Zusammensetzung nach einem der Ansprüche 21 bis 31 auf die Keratinsubstanzen umfasst.

**33.** Kosmetisches Verfahren zum Schminken der Haut des Gesichts und/oder der Lippen, das das Auftragen einer kosmetischen Zusammensetzung für Make-up oder Lippenstift auf die Keratinsubstanzen umfasst, wie sie in Anspruch 31 definiert sind.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9624620 A
- WO 0071501 A
- WO 9630421 A
- FR 2821620
- WO 9801478 A
- WO 9935177 A
- WO 9858974 A
- WO 9931144 A
- WO 9701478 A
- EP 895467 A
- EP 96459 A
- US 5625005 A


### Littérature non-brevet citée dans la description


- *Macromolecules,* 2001, vol. 34, 2667
- **Fischer.** *Chemical Reviews,* 2001, vol. 101, 3581
- **Tordo ; Gnanou.** *J. Am. Chem. Soc.,* 2000, vol. 122, 5929
- **Hawker.** *J. Am. Chem. Soc.,* 1999, vol. 121, 3904
- **Rizzardo.** *Macromolecules,* 1998, vol. 31, 5559
- **T. Pakula et al.** *Macromol. Theory Simul.,* 1996, vol. 5, 987-1006
- **A. Aksimetiev et al.** *J. of Chem. Physics,* vol. 111 (5
- **M. Janco.** *J. Polym. Sci., Part A: Polym. Chem.,* 2000, vol. 38 (15), 2767-2778
- **M. Zaremski et al.** *Macromolecules,* 2000, vol. 33 (12), 4365-4372
- **K. Matyjaszewski.** *J. Phys. Org. Chem.,* 2000, vol. 13 (12), 775-786
- **Gray.** *Polym. Prepr. (Am. Chem. Soc., Div. Polym. Chem.),* 2001, vol. 42 (2), 337-338
- **K. Matyjaszewski.** *Chem. Rev.,* 2001, vol. 101 (9), 2921-2990
- Polymer Handbook. John Wiley, 1989
- Solubility parameter values. **Eric A.Grulke.** Polymer Handbook. 519-559
- **C.M.Hansen.** The three dimensional solubility parameters. *J.Paint Technol.,* 1967, vol. 39, 105